# EUROPEAN PATENT SPECIFICATION

(11) **EP 3 722 313 B1**
(45) Date of publication and mention of the grant of the patent: **04.11.2026**
(21) Application number: 18886531.5
(22) Date of filing: 06.12.2018
(51) Int. Cl.: C07K 16/28, C07K 14/725, C12N 5/0783, A61K 35/17, A61P 35/00

(54) **ANTIBODY OR ANTIGEN BINDING FRAGMENT THEREOF FOR SPECIFICALLY RECOGNIZING B CELL MALIGNANCY, CHIMERIC ANTIGEN RECEPTOR COMPRISING SAME, AND USE THEREOF**
ANTIKÖRPER ODER ANTIGENBINDENDES FRAGMENT DAVON ZUR SPEZIFISCHEN ERKENNUNG VON B-ZELL-MALIGNITÄT, CHIMÄRER ANTIGENREZEPTOR DAMIT UND VERWENDUNG DAVON
ANTICORPS OU SON FRAGMENT DE LIAISON À L'ANTIGÈNE POUR RECONNAÎTRE SPÉCIFIQUEMENT UNE MALIGNITÉ DE CELLULE B, RÉCEPTEUR D'ANTIGÈNE CHIMÉRIQUE LE COMPRENANT, ET SON UTILISATION

(30) Priority: 06.12.2017 KR 20170166969
(43) Date of publication of application: 14.10.2020
(73) Proprietor: AbClon Inc., Seoul 08381 (KR)
(72) Inventor: LEE, Jong Seo, Seongnam-si Gyeonggi-do 13473 (KR); KIM, Kyu Tae, Goyang-si Gyeonggi-do 10551 (KR); KO, Bong Kook, Seoul 08766 (KR); KIM, Ki Hyun, Seoul 06783 (KR)
(74) Representative: J A Kemp LLP
(86) International application number: PCT/KR2018/015445
(87) International publication number: WO 2019/112347

(56) References cited:
- WO-A1-2014/184143
- WO-A1-2016/139487
- WO-A2-2017/066136
- CN-A- 106 554 414
- KR-A- 20150 132 850
- KR-A- 20170 057 298
- US-B2- 8 097 703
- US-B2- 8 679 492
- US-B2- 9 701 758
- KATZ BEN-ZION ET AL: "Therapeutic targeting of CD19 in hematological malignancies: past, present, future and beyond", LEUKEMIA & LYMPHOMA, INFORMA HEALTHCARE, UK, vol. 55, no. 5, 1 May 2014 (2014-05-01), pages 999 - 1006, XP009187786, ISSN: 1029-2403, DOI: 10.3109/10428194.2013.828354

## Description

### Technical Field

Described herein is a CD19-specific chimeric antigen receptor, an effector cell expressing said chimeric antigen receptor, a pharmaceutical composition comprising the effector cell, a nucleic acid molecule encoding the chimeric antigen receptor, and a recombinant vector comprising the nucleic acid molecule.

### Background Art

B cell malignancies are tumors generated in B cells, which are a type of cell lineage responsible for the immune system of the body. Such a B cell malignancy breaks a normal immune system to decrease the immunity against antigens invading from the outside, finally causing the death of patients. For example, acute lymphocytic leukemia (ALL), which is one of B cell malignancies, refers to a disease in which the lymphoid line of white blood cells becomes malignant, grows in the bone marrow, and spreads to peripheral blood, thus invading the liver, the spleen, the lymph, the cerebrum, the cerebellum, the spinal cord, and so on. Representative of therapies for acute lymphocytic leukemia are chemotherapy, targeted therapy, and allogeneic stem cell transplantation. These therapies have been improved to carry the survival rate of child patients to over 85 %. However, there are patients unresponsive to conventional therapies or patients in recurrence, and acute lymphocytic leukemia is the most common cause of cancer and death from cancer among children.

Most lymphoma/leukemia generated from B cell malignancies as well as acute lymphocytic leukemia are characterized by the expression of CD19 antigen on the surface of the cells. On the basis of this feature, various therapies designed to recognize CD19 antigen have been tried. Among such CD19 target therapies, CAR-T cell therapy was used for treatment of blood cancer through the cell death induction mechanism thereof as it was found to increase cytotoxicity for target cells in acute leukemia patients unresponsive to conventional therapies. A high cure rate (27 of 30 cases) was reported as a clinical test result of the therapy. However, in spite of the high response rate thereof, conventional CD19 CAR-T cell therapies were reported to have the problem of causing resistance in 10-20% of the patients treated therewith (Maude et al., N Eng J Med, 2014, 371:1507; Topp et al., J Clin Oncol, 2014, 32:4134). Therefore, there is a need for the development of a novel antibody that binds to a site different from those bound by conventional CD19 targeting antibodies.

Under the background, the present inventors developed an antigen-binding fragment that selectively recognizes CD19-expressing B cells among B cell malignancies and found that the developed antibody binds to CD19 at an epitope different from that targeted by the conventional antibody FMC63. In addition, cytotoxic T cells that express a chimeric antigen receptor comprising the developed antigen-binding fragment retain cytotoxicity.

WO 2014/184143 describes a CD19 specific chimeric antigen receptor and uses thereof. WO 2016/139487 describes a chimeric antigen receptor comprising a CD19-binding domain. WO 2017/066136 describes antibody agents specific for human CD19 and uses thereof. US 89097703 describes CD19 antibodies and their uses. US 8679492 describes humanized antibodies that bind to CD19 and their uses. KR 20170057298 describes CD19 antibodies and chimeric antigen receptors specific for CD19. US 9701758 describes an anti-CD19 scFv (FMC63) polypeptide. KR 20150132850 describes treatment of cancer using humanized anti-CD19 chimeric antigen receptor. CN 106544414 describes an anti-CD 19 human antibody and immunologic effector cell targeting CD19. Katz BZ, and Herishanu Y. 2014 (Leukemia & Lymphoma. 2014 May, Vol.55 NO.5 pages 999-1006) relates to the therapeutic targeting of CD19 in hematological malignancies.

### Summary of the Invention

The invention provides a CD19-specific chimeric antigen receptor, comprising:
(a) an extracellular domain comprising a humanized anti-CD19 antibody or an antigen-binding fragment thereof;
(b) a transmembrane domain; and
(c) an intracellular signaling domain,
wherein the anti-CD19 antibody or the antigen-binding fragment thereof comprises a heavy chain variable region and a light chain variable region comprising, respectively:
(i) the sequences of SEQ ID NOS: 7 and 8;
(ii) the sequences of SEQ ID NOS: 42 and 43;
(iii) the sequences of SEQ ID NOS: 46 and 47;
(iv) the sequences of SEQ ID NOS: 50 and 51;
(v) the sequences of SEQ ID NOS: 54 and 55;
(vi) the sequences of SEQ ID NOS: 58 and 59;
(vii) the sequences of SEQ ID NOS: 62 and 63;
(viii) the sequences of SEQ ID NOS: 66 and 67; or
(ix) the sequences of SEQ ID NOS: 70 and 71.

The invention further provides an effector cell, expressing the chimeric antigen receptor of the invention.

The invention also provides a pharmaceutical composition comprising the effector cell of the invention for preventing or treating a CD19 positive cell-associated disease, an autoimmune disease, or an inflammatory disease.

The invention also provides a nucleic acid molecule, encoding the chimeric antigen receptor of the invention.

The invention also provides a recombinant vector, comprising the nucleic acid molecule of the invention.

### Detailed Description

### Technical Problem

Leading to the present disclosure, intensive and thorough research into development of a novel antibody binding to a different epitope of CID19 and a chimeric antigen receptor using the same, conducted by the present inventors in order to solve the problem of resistance to conventional CD19-specific CAR-T therapies, resulted in the finding that CD19_12.18 antibody and variants thereof bind to a CD19 epitope site different from those that the conventional antibody FMC63 targets.

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

References to methods of treatment by therapy or surgery or in vivo diagnosis methods in the description are to be interpreted as references to compounds, pharmaceutical compositions and medicaments of the present invention for use in those methods.

According to an aspect thereof, the present disclosure provides an anti-CD19 antibody or an antigen-binding fragment thereof, comprising the following:
(a) a heavy chain variable region comprising the following heavy chain complementarity determining region (CDR) amino acid sequences: CDRH1 of SEQ ID NO: 1 and CDRH2 of SEQ ID NO: 2; and
(b) a light chain variable region comprising the following light chain CDR amino acid sequence: CDRL1 of SEQ ID NO: 4.

In an aspect of the present disclosure, the heavy chain variable region further comprises CDRH3 comprising any one of the amino acid sequences of SEQ ID NOS: 3 and 30 to 35.

In another aspect of the present disclosure, the light chain variable region further comprises CDRL2 comprising any one of the amino acid sequences of SEQ ID NOS: 5 and 36 to 39.

In another aspect of the present disclosure, the light chain variable region further comprises CDRL3 comprising any one of the amino acid sequences of SEQ ID NOS: 6, 40, and 41.

In another aspect of the present disclosure, the heavy chain variable region comprises any one of the sequences of SEQ ID NOS: 7, 42, 46, 50, 54, 58, 62, 66, and 70.

In another aspect of the present disclosure, the light chain variable region comprises any one of the amino acid sequences of SEQ ID NOS: 8, 43, 47, 51, 55, 59, 63, 67, and 71.

According to an aspect thereof, the present disclosure provides an anti-CD19 antibody or an antigen-binding fragment thereof which binds specifically to CD19 and shields CD19 from being bound by an antibody or an antigen-binding fragment thereof which binds to at least one amino acid residue selected from the group consisting of T51, S53, E55, L58, K59, and K63 on the amino acid sequence of SEQ ID NO: 92. The amino acid sequence of SEQ ID NO: 92 comes from human B lymphocyte antigen CD19 and is known as ID: P15391 in UniProtKB.

The antibody or the antigen-binding fragment of the present disclosure binds specifically to CD19 and shields CD19 from being bound by an antibody or an antigen-binding fragment thereof which binds to at least two amino acid residues selected from the group consisting of T51, S53, E55, L58, K59, and K63 on the amino acid sequence of SEQ ID NO: 92.

The antibody or the antigen-binding fragment of the present disclosure binds specifically to CD19 and shields CD19 from being bound by an antibody or an antigen-binding fragment thereof which binds to at least three amino acid residues selected from the group consisting of T51, S53, E55, L58, K59, and K63 on the amino acid sequence of SEQ ID NO: 92.

The antibody or the antigen-binding fragment of the present disclosure binds specifically to CD19 and shields CD19 from being bound by an antibody or an antigen-binding fragment thereof which binds to at least one amino acid residue selected from the group consisting of L58, K59, and K63 on the amino acid sequence of SEQ ID NO: 92.

The antibody or the antigen-binding fragment of the present disclosure binds specifically to CD19 and shields CD19 from being bound by an antibody or an antigen-binding fragment thereof which binds to at least two amino acid residues selected from the group consisting of L58, K59, and K63 on the amino acid sequence of SEQ ID NO: 92.

In an aspect of the present disclosure, the antibody or the antigen-binding fragment thereof specifically targets CD19 and binds to at least one amino acid residue selected from the group consisting of T51, S53, E55, L58, K59, and K63 on the amino acid sequence of SEQ ID NO: 92.

In another aspect of the present disclosure, the antibody or the antigen-binding fragment thereof specifically targets CD19 and binds to at least two amino acid residues selected from the group consisting of T51, S53, E55, L58, K59, and K63 on the amino acid sequence of SEQ ID NO: 92.

In another aspect of the present disclosure, the antibody or the antigen-binding fragment thereof specifically targets CD19 and binds to at least three amino acid residues selected from the group consisting of T51, S53, E55, L58, K59, and K63 on the amino acid sequence of SEQ ID NO: 92.

In another aspect of the present disclosure, the antibody or the antigen-binding fragment thereof specifically targets CD19 and binds to at least one amino acid residue selected from the group consisting of L58, K59, and K63 on the amino acid sequence of SEQ ID NO: 92.

In another aspect of the present disclosure, the antibody or the antigen-binding fragment thereof specifically targets CD19 and binds to at least two amino acid residues selected from the group consisting of L58, K59, and K63 on the amino acid sequence of SEQ ID NO: 92.

As confirmed in an example of the present disclosure, the anti-CD19 antibody of the present disclosure or an antigen-binding fragment thereof (e.g. CD19_12.18) exhibited decreased binding affinity for mtCD19(T51V), mtCD19(S53C), mtCD19(E55D), mtCD19(L58F), mtCD19(K59E), and mtCD19(K63N), which were modified from the hCD19 consisting of the amino acid sequence of SEQ ID NO: 92 at T51, S53, E55, L58, K59, and K63 residues, respectively. From the result, it was observed that the six amino acid residues function as important key residues responsible for the epitope site at which the anti-CD19 antibody or antigen-binding fragment of the present disclosure binds to CD19. Of them, the amino acid residues L58, K59, and K63 were found to be more important key residues responsible for the binding of the antibody or the antigen-binding fragment to CD19 as the binding affinity was significantly decreased for mutant hCD19 in which L58, K59, and K63 were modified. Therefore, the anti-CD19 antibody of the present disclosure or an antigen-binding fragment thereof binds to hCD19 including the amino acid sequence of SEQ ID NO: 92 at one, two, three or more amino acid residues selected from the group consisting of T51, S53, E55, L58, K59, and K63 on the sequence. In detail, the anti-CD19 antibody of the present disclosure or an antigen-binding fragment thereof binds to hCD19 including the amino acid sequence of SEQ ID NO: 92 at least one amino acid residue selected from the group consisting of L58, K59, and K63 on the sequence. As a result, the anti-CD19 antibody of the present disclosure or an antigen-binding fragment thereof shields CD19 from being bound by an antibody or an antibody-binding fragment thereof which binds to hCD19 including the amino acid sequence of SEQ ID NO: 92 at least one selected from the group consisting of T51, S53, E55, L58, K59, and K63 on the amino acid sequence. More particularly, the anti-CD19 antibody of the present disclosure or an antigen-binding fragment thereof shields CD19 from being bound by an antibody or an antigen-binding fragment thereof which binds to hCD19 including the amino acid sequence of SEQ ID NO: 92 at least one selected from the group consisting of L58, K59, and K63 on the amino acid sequence.

In an aspect of the present disclosure, CD19_12.18 antibody or a variant thereof according to the present disclosure binds to CD19 at an epitope different from that of FMC63, which is an antibody used in a chimeric antigen receptor. The antibody of the present disclosure and variants thereof target the same epitope or compete with each other for binding to the same epitope in CD19.

As will be demonstrated in the Examples below, the anti-CD19 antibody or antigen-binding fragment of the present disclosure was greatly lowered in binding affinity for mtCD19(T51V), mtCD19(S53C), mtCD19(E55D), mtCD19(L58F), mtCD19(K59E), and mtCD19(K63N), which are hCD19 including the amino acid sequence of SEQ ID NO: 92 modified at amino acid residues T51, S53, E55, L58, K59, and K63, respectively. However, the binding affinity was maintained intact in FMC63 antibody. In addition, the antibody of the present disclosure or an antigen-binding fragment thereof normally binds to mtCD19 (H218R/KSS) whereas FMC63 antibody shows poor binding affinity for mtCD19(H218R/KSS). Therefore, the anti-CD19 antibody of the present disclosure or an antigen-binding fragment thereof does not bind to the epitope to which FMC63 antibody binds.

In another Example, an ELISA plate was coated with a the CD19_12.18 antibody variant (i.e., CD19_1218.81 antibody) of the present disclosure to which CD19-ECD-Ck alone or in combination with CD19_1218 antibody was added, followed by competitive ELISA. CD19_1218 antibody competed with CD19_1218.81 antibody for binding to CD19-ECD-Ck. As a result, a low level of CD19-ECD-Ck bound by CD19_1218 antibody was detected. Therefore, the anti-CD19 antibody and a variant thereof bind to the same epitope or compete with each other for the same epitope.

In the present disclosure, the antibody or antigen-binding fragment may be a human antibody or a humanized entity. In addition, the antibody or antigen-binding fragment of the present disclosure may be scFv, but is not limited thereto.

In the present disclosure, the antibody or the antigen-binding fragment was constructed by CDR grafting in which complementarity determining regions (CDRs) of conventional antibodies developed in mice into a framework more stable than those of the conventional antibodies. "CDR grafting" is a representative technique for humanizing a non-human antibody in order to solve the problem that murine monoclonal antibodies are neutralized due to the immunogenicity thereof upon use in human antibodies. That is, CDR grafting is to graft a CDR region of an animal antibody to the framework of human antibodies.

An antibody according to an aspect of the present disclosure is CD19_12.18 antibody or a variant thereof. In detail, the variant of CD19_12.18 antibody is hzCD19_1218.81, hzCD19_1218.82, hzCD19_1218.81.12, hzCD19_1218.81.17, hzCD19 1218.81.52, hzCD19 1218.81.55, hzCD19 1218.81.64, or hzCD19_1218.81.79. Amino acid sequences and nucleotide sequences of CDRs and light chain variable regions or heavy chain variable regions of the variants are given in the specification and the sequence listing appended.

In the specification, "FMC63" antibody is an example of murine anti-CD19 monoclonal antibodies (Nicholson et al., Molecular Immunology, 34(16-17): 1157-1165 (1997)). Variable regions of FMC63 monoclonal antibody have been used in CAR tested in clinical trials (e.g., see [Kochenderfer et al., Nature Review Clinical Oncol., 10(5); 267-276 (2013); Porter et al., New Eng. J. Med., 365(8): 725-733 (2011); Kalos et al., Science Translational Medicine, 3(95): 95ra73 (2011); Kochenderfer et al., Blood, 116(20): 4099-4102 (2010); and Kochenderfer et al., Blood, 119(12): 2709-2720 (2012)]).

As used herein, the term "antibody" used in context of CD19 refers to an antibody specific for CD19 and is intended to encompass not only a whole antibody form, but also an antigen-binding fragment thereof.

A whole antibody includes two full length light chain and two full length heavy chains where each light chain is linked to the heavy chain by disulfide bonds. The heavy chain constant region is divided into isotypes of gamma (γ), mu (µ), alpha (α), delta (δ), and epsilon (ε) types, which are further subtyped into gamma1 (γ1), gamma2 (γ2), gamma3 (γ3), gamma4 (γ4), alpha 1 (α1), and alpha 2 (α2). The light chain constant region is divided into kappa (κ) and lambda (λ) types.

As used herein, the term "antigen-binding fragments" refers to a fragment retaining the function of binding to an antigen and includes Fab, F(ab'), F(ab')2, and Fv. Of them, Fab (fragment antigen binding) is composed of one constant and one variable domain of each of the heavy and the light chain, the constant domain of the heavy chain being the first constant domain (CH1), and thus contains one antigen-binding site. Fab' is different from Fab in that the former comprises a hinge region including at least one cysteine residue at the C-terminal of the CH1 domain of a heavy chain. F(ab')2 is produced by a disulfide bond formation between cysteine residues in the hinge region of Fab'. Fv is an antibody fragment composed only of variable regions of a heavy and a light chain, which may be produced by a recombinant technology disclosed in the art. In Fv (two-chain Fv), variable regions of a light and heavy chain are linked by a non-covalent bond, and in a single chain Fv, variable regions of a light and heavy chain are linked by a covalent bond through a peptide linker or it may form a dimer structure like a two chain FV through a direct linkage at the C-terminal. These antibody fragments can be obtained through a proteinase treatment (for example, a whole antibody may be treated with a papain to obtain Fab fragments or with pepsin to obtain F(ab')2 fragment) or preferably constructed using a recombinant DNA technology.

Herein, examples of the antibody include a monoclonal antibody, a multispecific antibody, a human antibody, a humanized antibody, a chimeric antibody, a single-chain Fv (scFv), a single-chain antibody, an Fab, an F(ab' ), a disulfide-linked Fv (sdFv), an anti-idiotype (anti-Id) antibody, and an epitope-binding fragment thereof, but are not limited thereto.

The term "heavy chain", as used herein, refers to a full length chain comprising three constant regions CH1, CH2 and CH3 and one variable region VH comprising an amino acid sequence which is sufficient for conferring specificity to an antigen as well fragments thereof. Also The term "light chain" as used herein refers to a full length chain comprising one constant region CL and one variable region VL comprising an amino acid sequence which is sufficient for conferring specificity to an antigen as well fragments thereof.

The term "variable region" or "variable domain", as used herein, refers to a domain on a heavy or a light chain of an antibody, which is responsible for binding the antibody to an antigen. Variable domains on the heavy and the light chain of a native antibody (VH and VL, respectively) are generally similar in structure and each include four conserved framework regions (FRs) and three hypervariable regions (HVRs) (Kindt et al., Kuby Immunology, 6th edition, W.H. Freeman and Co., page 91 (2007)).

As used herein, the term "CDR" (complementarity determining region) refers to an amino acid sequence of the hypervariable regions on the immunoglobulin heavy and light chains (Kabat et al., Sequences of Proteins of Immunological Interest, 4th Ed., U.S. Department of Health and Human Services, National Institutes of Health (1987)). Three CDRs are included in each of the heavy chain (CDRH1, CDRH2, and CDRH3) and the light chain (CDRL1, CDRL2, and CDRL3). CDRs provides important contact residues with which the antibody binds to an antigen.

As used herein, the term "framework" or "FR" refers to variable domain residues other than hypervariable region (HVR) residues. The FR of the variable domain generally consists of four FR domains: FR1, FR2, FR3, and FR4. Thus, the HVR and FR sequences generally appear in the following sequence in VH:
FRH1 (framework region 1 of heavy chain)-CDRH1 (complementarity determining region 1 of heavy chain)-FRH2-CDRH2-FRH3-CDRH3-FRH4.

Also, HVR and FR sequences in VL (or Vk) are arranged in the order as follows: FRL1 (framework region 1 of light chain)-CDRL1(complementarity determining region 1 of light chain)-FRL2-CDRL2-FRL3-CDRL3-FRL4.

By the term "specifically binding" or wordings relevant thereto, it is intended that an antibody or a constituent thereof, such as an antigen binding fragment or scFv, forms a complex with an antigen that is relatively stable under physiologic conditions. Specific binding can be characterized by an equilibrium dissociation constant of at least about 1x10⁻⁶ M or less (e.g., less KD means more strong binding). Methods for determining whether two molecules specifically bind are well known in the art and include, for example, equilibrium dialysis, surface plasmon resonance, and the like.

The term "affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein.

The term "human antibody", as used herein, refers to an antibody which possesses an amino acid sequence which corresponds to that of an antibody produced by a human or a human cell or derived from a non-human source that utilizes human antibody repertoires or other human antibody-encoding sequences.

The term "chimeric antibody" refers to an antibody in which a portion of the heavy and/or light chain is derived from a particular source or species, while the remainder of the heavy and/or light chain is derived from a different source or species.

In the context of specifically recognizing CD19, variants of the amino acid sequences listed in the sequence listing appended may fall within the scope of the anti-CD19 antibody or an antigen-binding fragment thereof according to the present disclosure. For example, a variation may be given to the amino acid sequence of an antibody in order to improve the binding affinity and/or other biological properties of the antibody. The variation includes a deletion, an addition, and/or a substitution of an amino acid residue on the amino acid sequence of the antibody.

Such amino acid variations may be provided on the basis of a relative similarity of amino acid side chains, e.g., hydrophobicity, hydrophilicity, charge, and size. As analyzed for size, shape, and type of amino acid side chains, it is clear that all of arginine, lysine and histidine residues are positively charged; alanine, glycine, and serine are similar in size; phenylalanine, tryptophan, and tyrosine have similar shapes. Accordingly, based on this consideration, arginine, lysine and histidine; alanine, glycine and serine; and phenylalanine, tryptophan and tyrosine may be considered to be biologically functional equivalents.

In making such variations, the hydropathic index of amino acids may be considered. Each amino acid has been assigned a hydropathic index on the basis of their hydrophobicity and charge characteristics: isoleucine (+4.5); valine (+4.2); leucine (+3.8); phenylalanine (+2.8); cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is generally understood in the art. It is known that similar biological activity is retained only upon substitution of certain amino acids for other amino acids having a similar hydropathic index. In making variations based upon the hydropathic index, the substitution of amino acids whose hydropathic indices are within ±2 is preferred, those which are within ±1 are particularly preferred, and those within ±0.5 are even more particularly preferred.

It also is understood in the art that substitutions between amino acids having similar hydrophilicity values may result in the generation of proteins having biologically equivalent activities.

As detailed in U.S. Pat. No. 4,554,101, the following hydrophilicity values have been assigned to amino acid residues: arginine (+3.0); lysine (+3.0); aspartate (+3.0±1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

In making variations based upon similar hydrophilicity values, the substitution of amino acids whose hydrophilicity values are within ±2 is preferred, those which are within ±1 are particularly preferred, and those within ±0.5 are even more particularly preferred.

The amino acid exchanges in proteins that do not substantially change the activity of the molecule are well known in the art (H. Neurath, R.L.Hill, The Proteins, Academic Press, New York, 1979). The most common exchanges are found between amino acid residues: Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly.

According to an aspect, the anti-CD19 antibody or the antigen-binding fragment of the present disclosure comprises: a heavy chain variable region containing at least one CDR including one amino acid sequence selected from SEQ ID NOS: 1 to 3 and SEQ ID NOS: 30 to 35; and a light chain variable region containing at least one CDR including one amino acid sequence selected from SEQ ID NOS: 4 to 6 and SEQ ID NOS: 36 to 41, and includes a monoclonal antibody, a multispecific antibody, a human antibody, a humanized antibody, a chimeric antibody, a single-chain Fv (scFv), a single-chain antibody, an Fab, an F(ab' ), a disulfide-linked Fv (sdFv), an anti-idiotype (anti-Id) antibody, and an epitope-binding fragment thereof, but are not limited thereto.

In another aspect, the anti-CD19 antibody or the antigen-binding fragment of the present disclosure comprises a heavy chain variable region including any one amino acid sequence of SEQ ID NOS: 7, 42, 46, 50, 54, 58, 62, 66, and 70.

In another aspect, the anti-CD19 antibody or the antigen-binding fragment of the present disclosure comprises a light chain variable region including any one amino acid sequence of SEQ ID NOS: 8, 43, 47, 51, 55, 59, 63, 67, and 71.

In another aspect, the anti-CD19 antibody or the antigen-binding fragment of the present disclosure is an anti-CD19 scFv.

In an exemplary aspect of the present disclosure, the heavy chain variable region and the light chain variable region contained in the antibody or the antigen-binding fragment thereof are connected to each other via (Gly-Ser)n, (Gly₂-Ser)n, (Gly₃-Ser) n or (Gly₄-Ser) n linker, wherein n is an integer of 1 to 6 and particularly 3 to 4, but is not limited thereto. The light chain variable region and the heavy chain variable region in scFv may be, for example, arranged as follows: light chain variable region-linker-heavy chain variable; or heavy chain variable region-linker-light chain variable region.

Being of very poor similarity to CDR sequences of conventional anti-CD19 antibodies or chimeric antigen receptors including the same, the CDR sequence of the antibody of the present disclosure is unique. For example, a BLAST search performed for CD19_12.18 antibody of the present disclosure on the NCBI website detected an antibody disclosed in U. S. Pat. No. 9,074,002 (SEQ ID NO: 29) as the most homologous antibody, but with the CDR sequence homology therebetween being just 81.7 %. Moreover, the antibody disclosed in U. S. Pat. No. 9,074,002 binds to protein tyrosine phosphatase 1B (PTP1B), which is different from the target of the antibody of the present disclosure.

According to another aspect thereof, the present disclosure provides a nucleic acid molecule encoding the anti-CD19 antibody or the antigen-binding fragment thereof.

According to an aspect of the present disclosure, the nucleic acid molecule encoding the anti-CD19 antibody or the antigen-binding fragment thereof comprises at least one CDR-encoding nucleotide sequence selected from the group consisting of the nucleotide sequences of SEQ ID NOS: 10 to 12 and at least one CDR-encoding nucleotide sequence selected from the group consisting of the nucleotide sequences of SEQ ID NOS: 13 to 15.

According to another aspect of the present disclosure, the nucleic acid molecule comprises a heavy chain variable region-encoding nucleotide sequence including a nucleotide sequence selected from the group consisting of SEQ ID NOS: 16, 44, 48, 52, 56, 60, 64, 68, and 72.

According to another aspect of the present disclosure, the nucleic acid molecule comprises a heavy chain variable region-encoding nucleotide sequence including a nucleotide sequence selected from the group consisting of SEQ ID NOS: 17, 45, 49, 53, 57, 61, 65, 69, and 73.

According to another aspect of the present disclosure, the nucleic acid molecule comprises a nucleotide sequence encoding the antibody or the antigen-binding fragment having SEQ ID NO: 18, but is not limited thereto.

The term "nucleic acid molecule", as used herein, is intended to encompass DNA (gDNA and cDNA) and RNA molecules. Nucleotides are the basic building block of the nucleic acid molecule and include sugar or base-modified analogues as well as natural nucleotides (Scheit, Nucleotide Analogs, John Wiley, New York (1980); Uhlman and Peyman, Chemical Reviews, 90:543-584(1990)) .

It should be understood to a person skilled in the art that the nucleotide sequence coding for the antibody, the antigen-biding fragment thereof, or the chimeric antigen receptor polypeptide according to the present disclosure is any nucleotide sequence that encode an amino acid sequence constituting the chimeric antigen receptor molecule and is not limited to particular nucleotide sequences.

The reason is that even if the nucleotide sequence undergoes mutation, the expression of the mutated nucleotide sequence into a protein may not cause a change in the protein sequence. This is called the degeneracy of codons. Therefore, the nucleotide sequence includes nucleotide sequences containing functionally equivalent codons, codons encoding the same amino acids (e. g., arginine or serine are six different codons), or codons containing biologically equivalent amino acids.

According to an aspect of the present disclosure, nucleotide sequences of nucleic acids coding for polypeptides of heavy chain CDRs, light chain CDRs, heavy chain variable regions, light chain variable regions, heavy chains, or light chains in the antibody to CD19 or the antigen-binding fragment thereof according to the present disclosure are listed in the sequence listing appended.

The nucleic acid molecule of the present disclosure which encodes the anti-CD19 antibody or the antigen-binding fragment thereof is construed to encompass nucleotide sequences having substantial identity to the nucleic acid molecule. In this context, the term "substantial identity" refers to an identity of at least 80 %, more preferably at least 90 %, and most preferably at least 95 % between the nucleotide sequence of the present disclosure and any other sequences as analyzed by commonly used algorithm when alignment there between is made as much correspondingly as possible.

Considering the above-described mutations having biologically equivalent activity, it should be construed that nucleic acid molecules encoding the antibody or the antigen-binding fragment; or the chimeric antigen receptor polypeptide according to the present disclosure also include sequences having substantial identity therewith. In this regard, the substantial identity refers to an identity of at least 61 %, more preferably at least 70 %, still more preferably 80 %, and most preferably at least 90 % between the nucleotide sequence of the present disclosure and any other sequences as analyzed by commonly used algorithm when alignment therebetween is made as much correspondingly as possible. Methods of alignment for sequence comparison are known in the art. Various methods and algorithms for alignment are disclosed in Smith and Waterman, Adv. Appl. Math. 2:482(1981); Needleman and Wunsch, J. Mol. Bio. 48:443(1970*);* Pearson and Lipman, Methods in Mol. Biol. 24: 307-31(1988); Higgins and Sharp, Gene 73:237-44(1988); Higgins and Sharp, CABIOS 5:151-3(1989); Corpet et al., Nuc. Acids Res. 16:10881-90 (1988); Huang et al., Comp. Appl. BioSci. 8:155-65(1992) and Pearson et al., Meth. Mol. Biol. 24:307-31(1994). The NCBI Basic Local Alignment Search Tool (BLAST; Altschul, et al., J. Mol. Biol. 215:403-10(1990)) is available from, for example, the NBCI (National Center for Biological Information), and can be used in connection with sequence analysis programs, such as blastp, blasm, blastx, tblastn and tblastx, on the Internet. The use of the program in comparing sequence similarity can be available on the BLAST help page at the NCBI website.

Another aspect of the present disclosure provides a recombinant vector comprising a nucleic acid molecule coding for the anti-CD19 antibody or the antigen-binding fragment thereof.

According to another aspect thereof, the present disclosure provides a host cell transformed with the recombinant vector.

So long as it allows a vector to be cloned thereto and expressed sequentially, any host cell can be used in the present disclosure. Such host cells are well known in the art. For example, eukaryotic host cells suitable for the vector include monkey kidney cells (COS7), NSO cells, SP2/0, Chinese hamster ovary (CHO) cells, W138, baby hamster kidney (BHK) cells, MDCK, myeloma cell lines, HuT 78 cells, and HEK-293 cells, but are not limited thereto.

According to another aspect thereof, the present disclosure provides a CD19-specific chimeric antigen receptor comprising the following:
(a) an extracellular domain containing the anti-CD19 antibody or the antigen-binding fragment thereof;
(b) a transmembrane domain; and
(c) an intracellular signaling domain.

As used herein, the term "chimeric antigen receptor (CAR)" refers to an artificially constructed hybrid protein or polypeptide in which an antigen-binding domain (e.g., single-chain variable fragment (scFv)) of an antibody is linked to a T-cell signaling or T-cell activating domain. Taking advantage of the antigen-binding function of a monoclonal antibody, chimeric antigen receptors give T cells the new ability to target a specific protein in a non-MHC-restricted manner. Non-MHC-restricted antigen recognition provides CAR-expressing T cells with an ability to recognize irrespective of antigen processing, thus avoiding main tumor escape mechanisms. In addition, when expressed in T cells, CAR does advantageously not dimerize with intrinsic T-cell receptor (TCR) alpha and beta chains.

The chimeric antigen receptor of the present disclosure comprises an extracellular domain containing an antibody induced against CD19, known as a B lymphocyte antigen, or against an antigen-binding fragment thereof. In the present disclosure, the antibody induced against CD19 or an antigen-binding fragment thereof is as defined above for the anti-CD19 antibody or the antigen-binding fragment thereof.

According to an aspect of the present disclosure, the chimeric antigen receptor of the present disclosure is expressed on cell surfaces. Hence, the chimeric antigen receptor may comprise a transmembrane domain. The transmembrane domain may be derived from natural or synthetic sources known in the art. By way of example, the transmembrane domain may be a transmembrane domain of the protein selected from the group consisting of alpha, beta, or zeta chains of T-cell receptor, CD27, CD28, CD3 epsilon, CD45, CD4, CD5, CD8 (CD8α), CD9, CD16, CD22, CD33, CD37, CD64, CD80, CD86, CD134, CD137, and CD154, but is not limited thereto.

According to an exemplary aspect of the present disclosure, the transmembrane domain is the CD8-derived hinge/transmembrane domain encoded by SEQ ID NO: 20.

The term "intracellular signaling domain", as used herein, refers to a functional protein domain that produces a 2^{nd} messenger or functions as an effector in response to the 2^{nd} messenger to intracellularly transfer information so as to regulate cellular activity via a defined signaling pathway.

According to another aspect of the present disclosure, the chimeric antigen receptor of the present disclosure may comprise an intracellular signaling domain. The intracellular signaling domain is responsible for intracellular signaling following the binding of extracellular ligand binding domain to the target (e.g., CD19) resulting in the activation of the immune cell and immune response. In other words, the signal transducing domain is responsible for the activation of at least one of the normal effector functions of the immune cell in which the chimeric antigen receptor is expressed. For example, the effector function of a T cell can be a cytolytic activity or helper activity including the secretion of cytokines. Preferred examples of signal transducing domain for use in a chimeric antigen receptor can be the cytoplasmic sequences of the T cell receptor and co-receptors that act in concert to initiate signal transduction following antigen receptor engagement, as well as any derivate or variant of these sequences and any synthetic sequence that has the same functional capability.

According to an exemplary aspect, the intracellular signaling domain of the chimeric antigen receptor is a domain derived from CD3ζ (CD3 zeta) chain.

According to a still further exemplary aspect, the domain derived from the CD3ζ (CD3 zeta) chain is a CD3ζ domain encoded by a nucleotide sequence including SEQ ID NO: 22.

According to another exemplary aspect of the present disclosure, the intracellular signaling domain of chimeric antigen receptor further comprises at least one costimulatory molecule selected from the group consisting of OX40 (CD134), CD2, CD27, CD28, CDS, ICAM-1, LFA-1(CD11a/CD18), and 4-1BB (CD137). The intracellular signaling domain may be obtained or derived from an intracellular signaling molecule and may include an entirety or a part of the molecule from which the intracellular signaling domain is derived, as well as the domain described above.

According to an exemplary aspect of the present disclosure, the costimulatory domain may be a functional signaling domain obtained from a protein selected from the group consisting of CD28, OX40, 4-1BB (CD137), and/or ICOS (CD278) and, more particularly, a functional signaling domain of CD28 and/or OX40.

According to another aspect of the present disclosure, the intracellular signaling domain is a functional signaling of 4-1BB, CD28, OX40, CD3 zeta, or a combination thereof. Most particularly, the intracellular signaling domain is a functional signaling domain of CD3 zeta.

According to a more particular aspect of the present disclosure, the costimulatory molecule including CD137 is a CD3ζ domain encoded by a nucleotide sequence including SEQ ID NO: 21.

The transmembrane domain and intracellular signaling domain in the chimeric antigen receptor of the present disclosure may be at least one combination selected from among the transmembrane domains and intracellular signaling domains described above. For example, the chimeric antigen receptor of the present disclosure may comprise the CD8α transmembrane domain and the intracellular signaling domains of CD28 and CD3ζ.

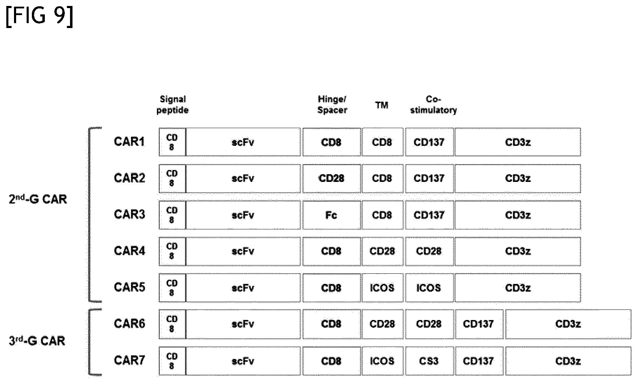

Structures of CAR constructs according to an aspect of the present disclosure are depicted in FIG. 9, with the amino acid/nucleotide sequences thereof given in the sequence listing appended.

Another aspect of the present disclosure provides a nucleic acid molecule encoding the chimeric antigen receptor described above.

The above-mentioned anti-CD19 antibody or the antigen-binding fragment (polypeptide) thereof, the nucleic acid molecule coding therefor, the chimeric antigen receptor comprising the anti-CD19 antibody or the antigen-binding fragment thereof, and the nucleic acid molecule coding for the chimeric antigen receptor are each in an isolated state.

As used herein, the term "isolated" means altered or removed from the natural/native state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

According to another aspect thereof, the present disclosure provides a recombinant vector comprising the above-mentioned nucleic acid molecule. For the "vector" to be described hereinafter, the antibody or the antigen-binding fragment thereof, or the nucleic acid molecule encoding a chimeric antigen receptor are commonly applied.

The term "vector" is intended to encompass a transfer vector and an expression vector.

As used herein, the term "transfer vector" refers to a composition of matter which comprises an isolated nucleic acid and which can be used to deliver the isolated nucleic acid into the interior of a cell. Examples of the transfer vector include, but are not limited to, linear polynucleotides, polynucleotides associated with ionic or amphiphilic compounds, plasmids, and viruses. More particularly, the transfer vector includes an autonomously replicating plasmid or virus. The term should also be construed to further include non-plasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector", as used herein, refers to a vector comprising a recombinant polynucleotide comprising expression control sequences operatively linked to a nucleotide sequence to be expressed in a host cell. An expression vector comprises sufficient cis-acting elements for expression; other elements for expression can be supplied by the host cell or in an in vitro expression system. Expression vectors include plasmids; cosmids; and viruses, such as bacteriophages, adenoviruses, lentiviruses, retroviruses, and adeno-associated viruses, which all incorporate the recombinant polynucleotide. According to an exemplary aspect of the present disclosure, a nucleic acid molecule coding for the switch molecule is operatively linked to a promoter in the vector of the present disclosure. As used herein, the term "operatively linked" means a functional linkage between a regulatory sequence for nucleic acid expression (example: a promoter, a signal sequence, or array of positions to which transcriptional factors bind) and other nucleic acid sequences, and by which the regulatory sequences are able to control the transcription and/or translation of the other nucleic acid sequence.

The recombinant vector system of the present disclosure can be constructed using various methods known in the art. With respect to concrete methods, reference may be made to Sambrook et al., Molecular Cloning, A Laboratory Manual, Cold Spring Harbor Laboratory Press (2001).

The vectors of the present disclosure may be constructed as a vector for gene cloning, for protein expression, or for gene transfer. Also, the vectors of the present disclosure may be constructed for eukaryotic or prokaryotic cells.

For example, when the present vector is an expression vector in a eukaryotic cell, promoters derived from genomes of mammalian cells (e.g., a metallothionein promoter, β-actin promoter, human hemoglobin promoter and human muscle creatinine promoter) or promoters derived from mammalian viruses (e.g., an adenovirus late promoter, a vaccinia virus 7.5K promoter, SV40 promoter, cytomegalovirus promoter, a tk promoter of HSV, a promoter of mouse mammary tumor virus (MMTV), a LTR promoter of HIV, a promoter of moloney virus, a promoter of Epstein Barr Virus (EBV), a promoter of Rous Sarcoma Virus (RSV)) may be use. Generally, the vectors include a polyadenylate sequence as a transcriptional termination sequence.

According to an aspect of the present disclosure, when used as a transfer vector, the vector may be "retroviral vector". Retroviruses provide a convenient platform for gene delivery systems. A selected gene can be inserted into a vector and packaged in retroviral particles. The recombinant virus can then be delivered to cells of the subject either in vivo or in vitro. A number of retroviral systems are known in the art. In some exemplary aspects, the retroviral vector may be a pMT retroviral vector, which is an MLV-based retroviral vector, but is not limited thereto.

According to an aspect of the present disclosure, the vector may be a lentivirus vector or an adenovirus vector.

The recombinant vector of the present disclosure may be fused with additional nucleotide sequences to facilitate the isolation and purification of the polypeptide expressed from the vector. The nucleotide sequences to be fused with the present vector include, for example, glutathione S-transferase (Pharmacia, USA), maltose binding protein (NEB, USA), FLAG (IBI, USA) and 6× His (hexahistidine; Qiagen, USA) and the like. The expression vector of the present disclosure may also comprise a selectable marker gene and/or a reporter gene as a selection marker for evaluating the expression of the antibody or the antigen-binding fragment and the CAR polypeptide containing the antibody. The selectable marker gene may be an antibiotic resistant gene typically used in the art, examples of which include genes resistant to ampicillin, gentamicin, carbenicillin, chloramphenicol, streptomycin, kanamycin, geneticin, neomycin, and tetracycline. The reporter gene may be exemplified by luciferase, beta-galactosidase, chloramphenicol acetyl transferase, and green fluorescent protein genes.

Methods of introducing and expressing genes into a cell are known in the art. In the context of an expression vector, the vector can be readily introduced into a host cell, e.g., mammalian, bacterial, yeast, or insect cell by any method known in the art. For example, the expression vector can be transferred into a host cell by physical, chemical, or biological means. The physical means include calcium phosphate precipitation, lipofection, particle bombardment, microinjection, electroporation, and the like. The chemical means include colloidal dispersion systems, such as macromolecule complexes, nanocapsules, microspheres, beads, and lipid-based systems including oil-in-water emulsions, micelles, mixed micelles, and liposomes. Within the biological means are the use of DNA or RNA vectors such as lentivirus, retrovirus, and the like.

According to another aspect thereof, the present disclosure provides a cell expressing the chimeric antigen receptor.

In one aspect of the present disclosure, the cell refers to a cell of hematopoietic origin functionally involved in the initiation and/or execution of an innate and/or adaptive immune response.

The immune cell according to the present disclosure may be derived from a stem cell. The stem cells may be adult stem cell, non-human embryonic stem cells, cord blood stem cells, bone marrow stem cells, induced pluripotent stem cells, or hematopoietic stem cells. More particularly, the immune cells may be selected from the group consisting of dendritic cells, killer dendritic cells, mast cells, NK-cells, B-cells or inflammatory T-lymphocytes, cytotoxic T-lymphocytes, regulatory T-lymphocytes, and helper T-lymphocytes, but are not limited thereto.

In the present disclosure, the chimeric antigen receptor-expressing cells are called effector cells. The effector cells include a population of autologous or allogeneic cells. In other words, the effector cells include a population of autologous or allogeneic cells expressing CAR specific for CD19.

According to an aspect of the present disclosure, the effector cells include a population of cells transduced or transfected with a vector comprising a nucleic acid molecular coding for a CD19-specific CAR. The transfection or transduction can be achieved by various means known in the art as described above, without limitations.

Hence, according to an exemplary aspect of the present disclosure, after being delivered into the effector cells, e.g., T lymphocytes or natural killer cells, the nucleic acid molecule coding for the CD19-specific CAR is transcribed into mRNA from which a CD19-specific CAR polypeptide is then translated, and expressed on the cell surface.

Also, another aspect of the present disclosure provides a pharmaceutical composition comprising a cell expressing the chimeric antigen receptor of the present disclosure.

The pharmaceutical composition may be provided in the form of a pharmaceutical composition comprising a chimeric antigen receptor-expressing cell and a pharmaceutically acceptable carrier.

When administered in the form of a pharmaceutical composition, the cell expressing the chimeric antigen receptor of the present disclosure may be a cell derived from an animal allogenic to the subject, or a cell autologous cell.

The pharmaceutical composition of the present disclosure comprises a population of cells expressing the chimeric antigen receptor of the present disclosure.

The pharmaceutical composition of the present disclosure comprises a cell expressing the chimeric antigen receptor of the present disclosure as an effective ingredient. Accordingly, the common descriptions between them are omitted in order to avoid undue redundancy leading to the complexity of this specification.

As will be proven in the following Example, when the chimeric antigen receptor T cells (CD19_12.18 CAR-T cells) containing the CD19_12.18 antibody fragment of the present disclosure and a CD19 antigen-expressing cell line (RaJi) are co-cultured, the CD19 antigen on the surface of the CD19-positve cell line (RaJi) is recognized to induce the activation of the chimeric antigen receptor. Thus, the pharmaceutical composition of the present disclosure is expected to find advantageous applications in the treatment of CD19 antigen-related diseases.

Diseases that can be prevented or treated by the pharmaceutical composition of the present disclosure are human and mammalian diseases associated with CD19 positive cells, including B cell malignancy selected from the group consisting of chronic lymphocytic leukemia (CLL), acute lymphocytic leukemia (ALL), pro-lymphocytic leukemia, hairy cell leukemia, common acute lymphocytic leukemia (CALLA), null-acute lymphoblastic leukemia, non-Hodgkin's lymphoma, diffuse large B cell lymphoma (DLBCL), multiple myeloma, follicular lymphoma, splenic lymphoma, marginal zone lymphoma, mantle cell lymphoma, indolent B cell lymphoma, and Hodgkin's lymphoma.

In addition, the diseases include autoimmune diseases and inflammatory diseases associated with inappropriate or enhanced B cell count and/or activation. Examples of the autoimmune diseases and inflammatory diseases include multiple sclerosis, rheumatoid arthritis, and systemic lupus erythematosus (SLE).

The pharmaceutically acceptable carrier contained in the pharmaceutical composition of the present disclosure is ordinarily used at the time of formulation, and examples thereof may include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present disclosure may further comprise a lubricant, a wetting agent, a sweetening agent, a flavoring agent, an emulsifier, a suspending agent, a preservative, and the like in addition to the above ingredients. Suitable pharmaceutically acceptable carriers and preparations are described in detail in Remington's Pharmaceutical Sciences (19th ed., 1995).

The pharmaceutical composition of the present disclosure may be administered orally or parenterally, for example, by intravenous injection, subcutaneous injection, intramuscular injection, intraperitoneal injection, intrasternal injection, intratumoral injection, topical administration, intranasal administration, intrapulmonary administration, and rectal administration.

The appropriate dose of the pharmaceutical composition of the present disclosure varies depending on factors, such as a formulating method, a manner of administration, patient's age, body weight, gender, and morbidity, food, a time of administration, a route of administration, an excretion rate, and response sensitivity. An ordinarily skilled practitioner can easily determine and prescribe an effective dose for desired treatment or prevention. According to a preferable aspect of the present disclosure, the daily dose of the pharmaceutical composition of the present disclosure is 0.0001-100 mg/kg. As used herein, the term "pharmaceutically effective amount" refers to an amount sufficient to prevent or treat the above-described diseases.

As used herein, the term "prevention" refers to a prophylactic or protective treatment of a disease or a disease condition. As used herein, the term "treatment" refers to a reduction, suppression, relief, or eradication of a disease condition.

The pharmaceutical composition of the present disclosure may be formulated into a unit dosage form or may be prepared in a multi-dose container by using a pharmaceutically acceptable carrier and/or excipient according to a method that can be easily implemented by a person having an ordinary skill in the art to which the present disclosure pertains. Here, the formulation may be in the form of a solution in an oily or aqueous medium, a suspension, an emulsion, an extract, a pulvis, a suppository, a powder, a granule, a tablet, or a capsule, and may further contain a dispersant or a stabilizer.

The pharmaceutical composition of the present disclosure may further comprise other pharmaceutically active agents or drugs, for example, chemotherapeutic agents such as asparaginase, busulfane, carboplatin, cisplatin, daunorubicin, doxorubicin, fluorouracil, gemcitabine, hydroxyurea, methotrexate, paclitaxel, rituximab, vinblastine, vincristine, and the like; targeted therapeutic agents such as bevacizumab, olaparib, and the like; or immune checkpoint inhibitors such as nivolumab, pembrolizumab, and the like, in addition to the above-described chimeric antigen receptor-expressing cells, or may be administered in combination therewith.

According to another aspect thereof, the present disclosure provides a method for treatment of a CD19 positive cell-associated disease, an autoimmune disease, or an inflammatory disease, the method comprising a step of administering to a subject in need thereof a composition comprising the antibody against CD19 or the antigen-binding fragment thereof; or a composition comprising an effector cell expressing the CD19-specific chimeric antigen receptor.

The CD19 positive cell-associated disease, the autoimmune disease, or the inflammatory disease, which are target diseases to be treated by the treated method, is as defined above for the target diseases of the pharmaceutical composition.

In an aspect of the present disclosure, the subject is a mammalian animal or a human.

Since the method for the prevention or treatment of cancer or inflammatory disease according to the present disclosure employs the above-described antibody or antigen-binding fragment; or the chimeric antigen receptor-expressing effector cell as an effective ingredient, the overlapping descriptions thereof are omitted to avoid excessive complexity of the specification

### Advantageous Effects

The antibody of the present disclosure binds specifically to CD19 that is highly expressed in cancer cells (particularly, blood cancer) and is very poor in CDR sequence homology to conventional CD19 target antibodies. Thus, the antibody of the present disclosure has a characteristic sequence which leads to specifically binding to an epitope different from that for conventional FMC63 antibody fragments. Inducing immune cell activation in response to stimulation with CD19-positive cells, the anti-CD19 antibody or the antigen-binding fragment of the present disclosure can be advantageously used as a CAR-immune cell therapy product.

### Brief Description of the Drawings

FIG. 1 is a graph illustrating the binding of CD19_12.18 antibody fragment to CD19-ECD protein as analyzed by ELISA.
FIG. 2 shows histograms of binding affinity of CD19_12.18 antibody fragment for CD19-positve RaJi, RS4;11 cells and CD19-negative Jurkat cells as measured by flow cytometry.
FIG. 3 is a view illustrating comparison of epitopes between the developed antibody fragment and the FMC63 antibody fragment. For epitope comparison with FMC63, FMC63 and CD19-ECD proteins are immobilized to a sensor chip to which the CD19_12.18 antibody fragment of the present disclosure is then applied.
FIG. 4 is a bar graph showing the identification of an epitope for the developed antibody fragments as measured by flow cytometry. The developed antibody fragments were applied to 293 cells in which mutant CD19 had been expressed through transient transfection, with FMC64 antibody serving as a control.
FIG. 5 is a bar graph identifying the binding of FMC63 and the developed antibody fragments to the epitope reported for FMC63. Epitope identification was conducted with 293 cells in which mutant CD19 had been expressed through transient transfection.

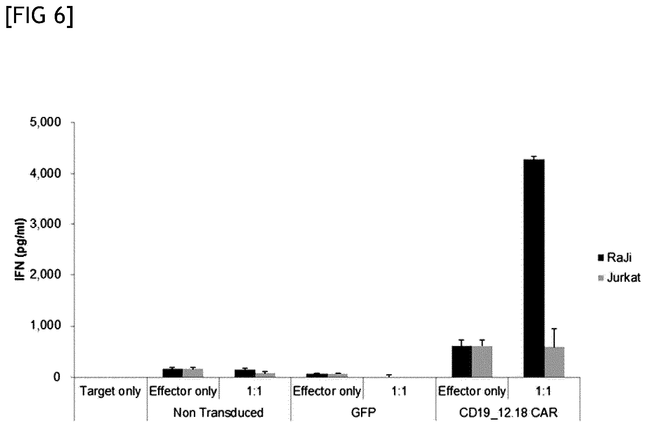

FIG. 6 is a bar graph showing activity of cytotoxic T cells expressing the chimeric antigen receptors conjugated with the antibody fragments of the present disclosure, as measured for secretion levels of interferon gamma.
FIG. 7 is a plot showing cell binding potentials of antibody fragments developed through affinity improvement and humanization. CD19-positive RaJi cells were used for the analysis [unit: MFI (mean fluorescence intensity)].
FIG. 8a is a bar graph showing activity of cytotoxic T cells expressing the chimeric antigen receptors conjugated with the antibody fragments of the present disclosure, as measured for secretion levels of interferon gamma. CD19-positive RaJi-Luc cells and cytotoxic T cells were co-cultured at a ratio of 1:5, followed by measuring levels of interferon gamma in the cell cultures. FIG. 8b shows cytotoxicity of cytotoxic T cells as measured for luciferase activity of RaJi-Luc cells surviving after co-culture with RaJi-Luc cells and cytotoxic T cells were co-cultured.
FIG. 9 is a view showing configurations of 7 constructs in which the chimeric antigen receptor components hinge region, transmembrane domain, and costimulatory domain were modified to optimize the activity of the developed antibody fragments.
FIG. 10 showing expression of the 7 modified chimeric antigen receptors as analyzed by flow cytometry. CD3 was used as a marker for analyzing the expression of the 7 modified chimeric antigen receptors in cytotoxic T cells.
FIG. 11a is a bar graph showing activity of cytotoxic T cells expressing 7 chimeric antigen receptors as measured for levels of interferon gamma. CD19-positive RaJi cells and CD19-negative Jurkat cells were used as target cells and each co-cultured at a ratio of 1:5 with cytotoxic T cells, followed by measuring levels of interferon gamma. FIG. 11b is a plot showing activity of cytotoxic T cells as measured for luciferase activity of RaJi-Luc surviving after co-cultivation of RaJi-Luc cells and cytotoxic T cells.
FIG. 12a shows results of the octet test to identify that CD19_1218, CD19_1218.81, CD19_1218.81.79, and CD19_1218.82 antibodies bind to epitope sites different from those to which FMC63 binds. FIG. 12b shows results of competition ELISA using CD19_1218 and CD19_1218.81 antibodies. Relative binding is given when absorbance upon the absence of the competitor (CD19-ECD-Ck alone) is set forth as 100%.

### EXAMPLES

A better understanding of the present disclosure may be obtained through the following examples which are set forth to illustrate, but are not to be construed as limiting the present disclosure.

### EXAMPLE 1: Development of Antibody to CD19

For antibody development, an extracellular domain (ECD) of human CD19 protein was produced using animal cells. A DNA construct in a form where the C-terminal of ECD was conjugated to the hinge and Fc region (CH₂-CH₃) of human IgG1 (CD19-ECD-Fc) or to His tag (CD19-ECD-His) was cloned into pCEP4 (Invitrogen, Cat. No. V044-50), using the restriction enzymes Hind-III and BamH-I. Subsequently, the transient transfection of the cloned vector into FreeStyle 293F cells (Invitrogen, Cat. No. R790-07) was conducted using polyethyleneimine (Polyscience Inc., Cat. No. 23966), followed by purification from the cell culture with the aid of protein-A Ceramic HyperD F resin (PALL, Cat No. 20078-028) or Ni-NTA Superflow (Qiagen, Cat No. 30410). The purified protein was quantitated using Protein assay dye (Bio-Rad, Cat. No. 500-0006) and subjected to SDS-PAGE, followed by coomassie blue staining to determine concentration and purity. The CD19-ECD-His protein thus obtained was subcutaneously injected to chickens. From the immunized chicken, the spleen and the bursa were excised. Total RNA was extracted from the spleen and the bursa, using TRI reagent (Invitrogen, USA), and used to synthesis cDNA therefrom. A library of antibody fragments was constructed using well-known primers specific for variable regions of immunoglobulin heavy and light chains (see Table 1, Phage display: a laboratory manual, Carlos Barbas III, et al., Cold Spring Harbor Laboratory Press).

**TABLE 1**

| Primer Used for Construction of Antibody Fragment Library | | |
|---|---|---|
| Primer | Sense | Antisense |
| Primer for heavy chain variable region | 5′GGTCAGTCCTCTAGATCTTCCGG CGGTGGTGGCAGCTCCGGTGGTGGC GGTTCCGCCGTGACGTTGGACGAG 3′(SEQ ID NO: 24) | 5′CTGGCCGGCCTGGCC ACTAGTGGAGGAGACGA TGACTTCGGTCC 3′(SEQ ID NO: 25) |
| Primer for light chain variable region | 5′GTGGCCCAGGCGGCCCTGACTCA GCCGTCCTCGGTGTC 3′(SEQ ID NO: 26) | 5′GGAAGATCTAGAGGA CTGACCTAGGACGGTCA GG 3′(SEQ ID NO: 27) |
| Overlapping PCR primer | 5′GAGGAGGAGGAGGAGGAGGTGGC CCAGGCGGCCCTGACTCAG 3′(SEQ ID NO: 28) | 5′GAGGAGGAGGAGGAG GAGGAGCTGGCCGGCCT GGCCACTAGTGGAGG 3′(SEQ ID NO: 29) |

The chicken immune library thus constructed was subjected to phage bio-panning, with the CD19-ECD-Fc serving as an antigen. For use in bio-panning, the antibody library was obtained in a phage library form using VCSM13 helper phages. Up to four panning rounds were performed. For a panning strategy of enriching phages of high affinity, a lower amount of the antigen was used and a larger number of washing was conducted in a higher number of panning. The number of phages captured by the target antigen was tittered using ER2537 *E*. *coli* (New England Biolabs, Cat. No. 801-N) as follows. Binder phages obtained in each bio-panning round were eluted with glycine buffer at pH 2.2. The ER2537 *E. coli* was cultured overnight in SB (super broth) medium and then diluted by 1/200 in fresh SB medium before passage. Subsequently, an additional incubation for 3 hours at 37°C reached a log phage. In a 1.5-ml tube, 100 µl of fresh ER2537 *E. coli* and 10 µl of diluted phages were mixed and incubated for 30 min before being spread on ampicillin-containing LB (lysogeny broth) agar plates. After incubation overnight at 37°C, the number of phages was measured by applying the number of colonies thus formed and the dilution factor.

The binder phages obtained in each bio-panning round 2 were infected into ER2537 *E. coli.* While the bacteria were maintained in the colony form, ELISA was performed to examine binding to the antigen. To this end, first, the colonies obtained following phage infection were inoculated into SB medium and cultured until the OD₆₀₀ reached 0.5. Subsequently, the cell culture was incubated at 30°C in the presence of 0.5 mM IPTG while shaking so as to overexpress the antibody fragment proteins. Antibodies binding specifically to CD19 were selected by ELISA using CD19-ECD-Fc protein and by flow cytometry using Raji cells, which overexpress CD19. Through these methods, selection was made of CD19_12.18 that exhibited the highest binding affinity for human CD19. Amino acid sequences of the variable regions in the selected CD19_12.18 antibody are given in Table 2, below.

**TABLE 2**

| Amino Acid Sequence of CDR (Complementarity Determining Region) in CD19_12.18 Antibody | | |
|---|---|---|
| classification | light chain | heavy chain |
| CDR1 | SGGYSSYYG (SEQ ID NO: 4) | SYDMG (SEQ ID NO: 1) |
| CDR2 | ESNKRPS (SEQ ID NO: 5) | GIDDDGRYTSYGSAVDG (SEQ ID NO: 2) |
| CDR3 | GGWDSTHAGI (SEQ ID NO: 6) | GNAGWIDA (SEQ ID NO: 3) |

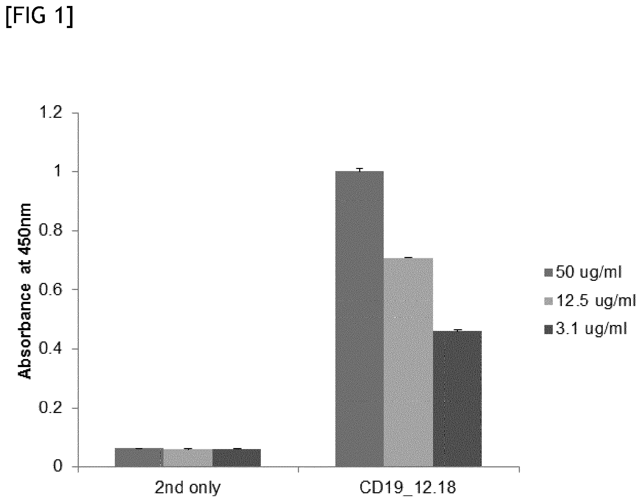

In order to quantitatively analyze the affinity of the selected CD19_12.18 antibody, antibody fragments including the variable regions were produced using animal cells. A DNA construct in a form where the C-terminal of ECD was conjugated to the hinge and Fc region (CH₂-CH₃) of human IgG1 (CD19-ECD-Fc) or to His tag (CD19-ECD-His) was cloned into pCEP4 (Invitrogen, Cat. No. V044-50). Subsequently, the cloned vector was transiently transfected into FreeStyle^{™} 293F cells (Invitrogen, Cat. No. R790-07). From the cell culture, the antibody in the Fc fusion protein form (Anti-CD19 scFv-Fc) was obtained. ELISA was conducted using CD19-ECD kappa light chain fusion protein (CD19-ECD-Ck) as a coating antigen so as to measure the binding affinity of the selected antibody. The purified antibody fragment (Anti-CD19 scFv-Fc) was applied at various concentrations (50, 12.5, and 3.1 µg/mL) to CD19-ECD protein-coated plates. Following incubation with a secondary antibody (anti-human Fc HRP), color was developed with TMB. OD₄₅₀ values were read on an ELISA reader (Victor X3 PerkinElmer) (FIG. 1). As shown in FIG. 1, CD19_12.18 antibody of the present disclosure was identified to bind specifically to CD19-ECD protein.

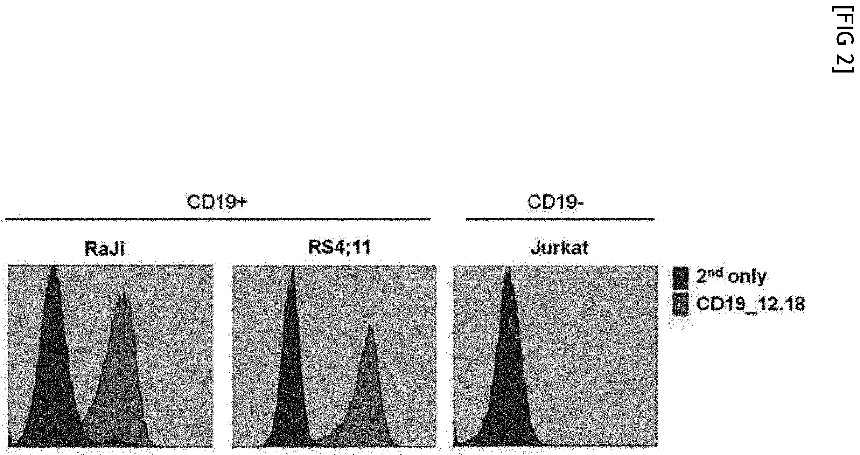

In addition, CD19_12.18, which binds to CD19-ECD protein was examined for affinity for the CD19 positive cell lines RaJi and RS4;11 and the CD19 negative cell line Jurkat. The CD19 positive cell lines RaJi and RS4;11 and the CD19 negative cell line Jurkat were treated with the purified antibody fragment (Anti-CD19 scFv-Fc). The antibody fragments bound to the cell lines were stained with anti-human IgG-FITC. Antibody fragments bound to the cell lines were measured by flow cytometry (FIG. 2). As can be seen in FIG. 2, CD19_12.18 antibody of the present disclosure was identified to be an antibody binding specifically to CD19 positive cells.

### EXAMPLE 2: Comparison of Epitopes between Developed Antibody Fragment and FMC63

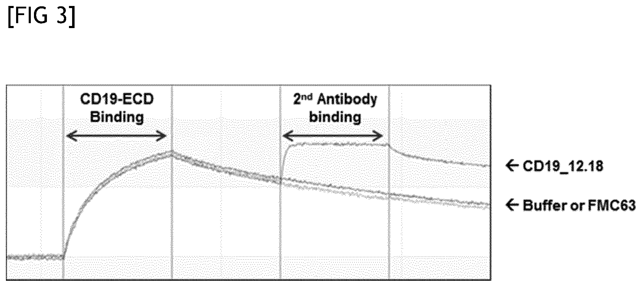

In order to examine whether the developed antibody has an epitope in common with FMC63, which is a mouse-derived CD19 antibody used in a chimeric antigen receptor (CAR) for treatment of B cell malignancy blood cancer, epitope binning was conducted using Octet system (Pall ForteBio). FMC63-Fc was fixed at a concentration of 10 µg/mL to AR2G sensor chip (Fortebio, Cat. No. 18-5092(tray), 18-5093(pack), 18-5094(case)) by an amine coupling method using EDC/NHS. The CD19-ECD kappa light chain fusion (CD19-ECD-Ck) was conjugated at a concentration of 10 µg/mL for 10 min to the FMC63-fixed sensor chip, followed by stabilizing the linkage between FMC63 and CD19-ECD for 5 min. Thereafter, CD19_12.18 antibody of the present disclosure or FMC63 was conjugated at a concentration of 10 µg/mL for 10 min, after which the linkage between the antigen and the antibody was stabilized for 10 min. Following fixation of FMC63, all the antibodies/antigen were diluted using kinetics buffer (Fortebio, cat No. 18-1092). The same buffer was also used for the stabilization step. In the case where the secondarily bound antibody further binds to the FMC63-bound CD19-ECD protein, the antibody can be construed to have no epitopes in common with FMC63. As shown in FIG. 3, FMC63 did not further bind whereas the CD19_12.18 antibody developed by the present inventors was observed to further bind to the FMC63-bound CD19-ECD. Therefore, CD19_12.18 antibody of the present disclosure is different from FMC63 antibody in terms of epitope.

### EXAMPLE 3: Identification of Epitope for the Developed Antibody

In order to identify epitopes therefor, the developed antibody was analyzed for binding to various mutant CD19 proteins constructed, using flow cytometry. In brief, first, the expression of CD19 protein was identified. To this end, the GFP protein-coexpressing bi-cistronic expression system (mutant CD19-T2A-GFP) using T2A system was digested with ClaI/XhoI and ligated to the pLenti6-V5/DEST lentiviral vector (Invitrogen, USA). The constructs thus obtained were analyzed by base sequencing. An examination was made of the binding of the antibody to the CD19 by flow cytometry for the 293 cell line which had undergone transient transfection to express the full-length CD19 protein and then treated with the purified antibody fragment (Anti-CD19 scFv-Fc).

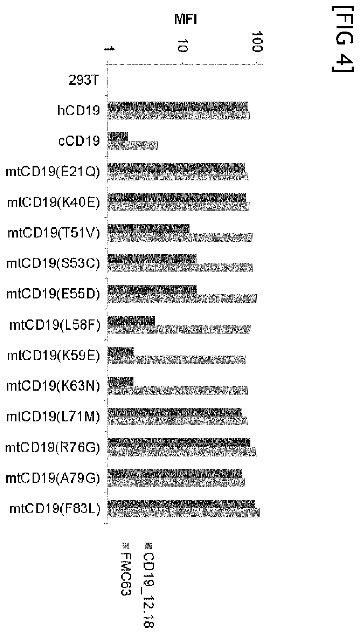

To begin with, the developed antibody was measured for binding affinity for recombinant human CD19 (hCD19, UniProtKB: P15391, SEQ ID NO: 92) and cynomolgus monkey CD19 (cCD19, UniprotKB: G7Q0T7, SEQ ID NO: 93). Like FMC63, the developed antibody was observed to have no cross-reactivity with cCD19 cross-reactivity (FIG. 4). For use in investigating epitopes for the developed antibody, mutant CD19 (mtCD19) proteins were made by substituting amino acids at specific positions with corresponding amino acids in cynomolgus monkey CD19. With respect to 12 amino acid residues different between hCD19 and cCD19 in sites other than already reported epitope sites for FMC63, mutant CD19 proteins having the amino acid residues of cCD19 were developed, followed by analyzing binding affinity therefor. Binding to GFP-positive cells was analyzed on the basis of mean fluorescence intensity (MFI). Of the 12 mutants tested, six residues (T51V, S53C, E55D, L58F, K59E, and K63N) were observed to play an important role in binding between the developed antibody CD19_12.18 and hCD19. Inter alia, the three mutants (L58F, K59E, and K63N) were found to completely suppress the binding of CD19_12.18 to the CD19, revealing the residues as key residues essential for the epitope to which the developed antibody binds (FIG. 4). In contrast, FMC63 was observed to bind intactly to the six mutant hCD19 proteins, indicating that the mutations do not influence the overall structure of hCD19, but alter the epitopic sites to which CD19_12.18 bind.

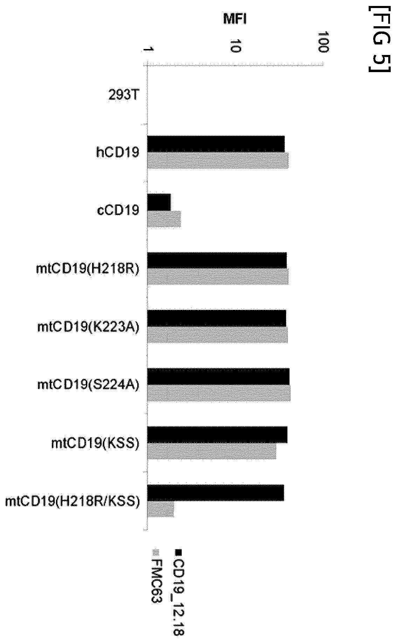

In addition, examination was made to see whether the antibody CD19_12.18 could bind to a mutant in which a site important for the binding of FMC63 thereto was mutated. In this regard, five mutants which had mutations made at sites important for the binding of FMC63 thereto were constructed (Sommermeyer D et al., Leukemia, 2017, 31(10):2191). As was consistent with the result of the reference document, it was observed that FMC63 exhibited altered binding affinity for only the mutant (H218R/KSS) in which the residue at position 218 was substituted with arginine and serine was inserted at position 224. In contrast, the developed antibody CD19_12.18 was observed to normally bind to the mutant, indicating that the antibody is different in epitope from FMC63 (FIG. 5).

### EXAMPLE 4: Preparation of Lentivirus Including Developed Antibody Fragment-Conjugated Chimeric Antigen Receptor

A chimeric antigen receptor was developed on the basis of the developed antibody CD19_12.18. For the chimeric antigen receptor, codon optimization was made of a CD8 leader, scFv-type CD19_12.18, a hinge and transmembrane domain of CD8, a cytoplasmic domain of CD137, and a cytoplasmic domain of CD3 zeta and the sequence thus optimized was digested with SpeI/XhoI before insertion into pLenti6-V5/DEST lentiviral vector (Invitrogen, USA). The construct thus obtained (SEQ ID NO: 23) was identified by base sequencing.

The prepared lentiviral construct was transduced, together with the plasmid pCMV-dR8.91 comprising viral coat protein VSV-G (vesicular stomatitis indiana virus G protein), gag, pol, and rev genes, into Lenti-X 293T cells (Takara Bio Inc., Japan). Transduction was performed using Lipofectamine 2000 (Invitrogen, USA) according to the manufacturer's protocol. Seventy-two hours after transduction, a lentivirus containing culture medium was enriched by 10 fold through a centrifugal filter (Millipore, USA).

### EXAMPLE 5: Preparation of T Cell Displaying Developed Antibody Fragment-Bearing Chimeric Antigen Receptor

Cytotoxic T cells on which CD19_12.18 antibody fragment (scFv)-bearing chimeric antigen receptors were displayed were prepared using the lentivirus obtained in Example 3.

First, human naive T cells were isolated and stimulated with Dynabeads^{™} Human T-Activator CD3/CD28 (Thermofisher scientific, USA) for 24 hours. Thereafter, the lentivius was infected for 24 hours into the cells in the presence of polybrene (Sigma-Aldrich, USA). Then, the medium was exchanged with a medium containing IL-2 (Gibco, USA), followed by incubation at 37°C in a 5% CO₂ atmosphere. The transduction of the CD19_12.18 fragment-bearing chimeric antigen receptor was analyzed by Western blotting using CD3z antibody (BD, USA), which accounts for a chimeric antigen receptor (FIG.4).

As shown in FIG. 4, the chimeric antigen receptor bearing the developed CD19_12.18 antibody fragment of the present disclosure was displayed on T cells. The T cells presenting the CD19_12.18-bearing chimeric antigen receptor on the surface thereof (CD19_12.18 CAR-T cells) were used in experiments within 24 hours after being prepared.

### EXAMPLE 6: Activity of Cytotoxic T Cell Presenting Developed Antibody Fragment-Bearing Chimeric Antigen Receptor on Surface thereof

The cytotoxic T cells presenting on the surface thereof the chimeric antigen receptor prepared in Example 4 (CD19_12.18 CAR-T cells) were used to examine whether the activation of the chimeric antigen receptor T cells is induced with the recognition of CD19 on cell surfaces.

Briefly, the CD19-positive cell line RaJi and the CD19-negative cell line Jurkat E6.1 were separately cultured in RPMI-1640 supplemented with 10% fetal bovine serum and 1% penicillin-streptomycin. First, the CD19-positive or negative cells were seeded at a density of 3x10⁴ cells/well into roundbottom 96-well plates. After removal of the culture supernatant, the prepared chimeric antigen receptor T cells (CD19_12.18 CAR-T cells) were added at a predetermined rate per well and incubated at 37°C for 24 hours in a 5% CO₂ atmosphere. Interferon gamma secreted to the medium was quantitated using an ELISA kit according to the manufacturer's protocol. The results are shown in FIG. 5. In this regard, a group in which chimeric antigen receptor T cells were added to plates containing target cells (Effector T cell only) and a group in which no chimeric antigen receptor T cells were added to plates containing target cells (Target cell only) were used as controls.

As can be seen in FIG. 5, significant increases in the secretion of interferon gamma were detected in the CD19_12.18 antibody fragment-bearing chimeric antigen receptor T cells of the present disclosure (CD19_12.18 CAR-T cells) and the CD19-positive cells (RaJi). Therefore, when recognizing CD19 on the CD19-positive cells (RaJi), the cytotoxic T cells (CD19_12.18 CAR-T cells) presenting the CD19_12.18 antibody fragment-bearing chimeric antigen receptor of the present disclosure on the surface thereof were induced to be activated.

### EXAMPLE 7: Improvement of Affinity of Developed Antibody Fragment and Development of Humanized Antibody

In order to acquire antibody fragments superior to CD19_12.18 in terms of binding affinity for CD19, heavy chain and light chain libraries were combined to produce new sub libraries. To this end, oligonucleotides having NNK degenerate codons were employed, with 70% or more of the sequence of CD19_12.18 maintained. A nucleic acid sequence coding for the CD19_12.18 antibody fragment was used as a template DNA. Random codons were incorporated into six CDRs by PCR. The antibody fragment amplicons were purified using QIAquick Gel Extraction Kit (QIAGEN, USA). The antibody fragment amplicons were ligated to pComb3XSS vector after both were digested with sfi I. The resulting recombinant vector was transduced into ER2537 to construct phage libraries. Antibodies were selected using the phage libraries in the same manner as in Example 1.

From the selected antibodies, humanized antibodies were developed by CDR grafting. For the human antibody to which the CDR of the developed antibody would be implanted, human germline V and J genes similar to each other in view of base sequence were selected using IMGT/V-QUEST (Brochet, X. et al., Nucl Acids Res. 36:503-508 (2008)). The developed humanized antibodies were produced in Fc tag forms, using FreeStyle^{™} 293F cells. IGHV3-74*01 and IGHJ5*01 were employed as V and J genes of the heavy chain, respectively. IGLV1-51*02 and IGLJ2*01 were employed as V and J genes of the light chain, respectively. Amino acid sequences of variable regions in heavy and light chains of the developed antibodies are given in Tables 3 and 4.

**TABLE 3**

| Amino Acid Sequence of Heavy Chain CDR Region of Antibody with Improved Affinity | | | |
|---|---|---|---|
| Antibody | 1^{st} Heavy chain | 2^{nd} Heavy chain | 3^{Rd} Heavy chain |
| hzCD19_1218.81 | SYDMG | GIDDDGRYTSYGSAVDG | GNAGWIDA |
| hzCD19_1218.82 | SYDMG | GIDDDGRYTSYGSAVDG | GNAGWIDA |
| hzCD19_1218.81.12 | SYDMG | GIDDDGRYTSYGSAVDG | GNAGWIST (SEQ ID NO: 30) |
| hzCD19_1218.81.17 | SYDMG | GIDDDGRYTSYGSAVDG | GNAGWIET (SEQ ID NO: 31) |
| hzCD19_1218.81.52 | SYDMG | GIDDDGRYTSYGSAVDG | GNAGWILT (SEQ ID NO: 32) |
| hzCD19_1218.81.55 | SYDMG | GIDDDGRYTSYGSAVDG | GNAGWIQN (SEQ ID NO: 33) |
| hzCD19_1218.81.64 | SYDMG | GIDDDGRYTSYGSAVDG | GNAGWIQT (SEQ ID NO: 34) |
| hzCD19_1218.81.79 | SYDMG | GIDDDGRYTSYGSAVDG | GNAGWIDH (SEQ ID NO: 35) |

**TABLE 4**

| Amino Acid Sequence of Light Chain CDR Region of Antibody with Improved Affinity | | | |
|---|---|---|---|
| Antibody | 1^{st} Light chain | 2^{nd} Light chain | 3^{rd} Light chain |
| hzCD19_1218.81 | SGGYSSYYG | ESNKRPS | GGLTPTHAGI (SEQ ID NO: 40) |
| hzCD19_1218.82 | SGGYSSYYG | ESNKRPS | GQSTRTHAGI (SEQ ID NO: 41) |
| hzCD19_1218.81.12 | SGGYSSYYG | ESDKRPA (SEQ ID NO: 36) | GGLTPTHAGI |
| hzCD19_1218.81.17 | SGGYSSYYG | ESNKRPS | GGLTPTHAGI |
| hzCD19_1218.81.52 | SGGYSSYYG | ETDKRPQ (SEQ ID NO: 37) | GGLTPTHAGI |
| hzCD19_1218.81.55 | SGGYSSYYG | ESGKRPA (SEQ ID NO: 38) | GGLTPTHAGI |
| hzCD19_1218.81.64 | SGGYSSYYG | ESQKRPL (SEQ ID NO: 39) | GGLTPTHAGI |
| hzCD19_1218.81.79 | SGGYSSYYG | ESNKRPS | GGLTPTHAGI |

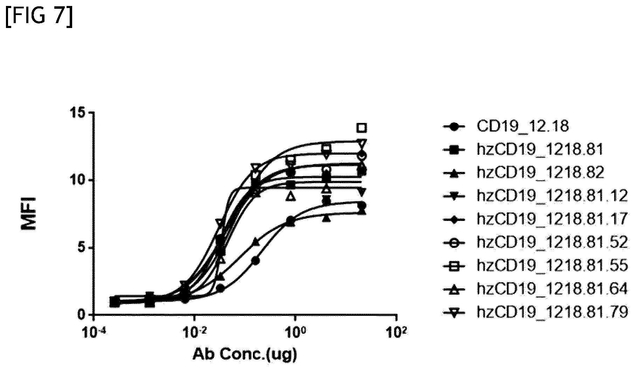

Following affinity improvement and humanization, the selected antibodies were assayed for binding affinity for the CD19-positive cell line RaJi. The CD19-positve cell line RaJi was incubated with various concentrations of purified antibody fragments, followed by staining with anti-human IgG-FITC. The antibody-bound RaJi cells were counted by flow cytometry (FIG. 7) and binding affinity was assayed by Graphpad Prism (Table 5). Through the assay, antibodies having higher binding potential than CD19_12.18 were secured.

**TABLE 5**

| Binding Potential of Affinity-Improved Antibody to RaJi Cell (EC₅₀) | |
|---|---|
| Antibody | EC₅₀ (µg) |
| CD19_12.18 | 0.213 |
| hzCD19_1218.81 | 0.032 |
| hzCD19_1218.82 | 0.078 |
| hzCD19_1218.81.12 | 0.034 |
| hzCD19_1218.81.17 | 0.038 |
| hzCD19_1218.81.52 | 0.038 |
| hzCD19_1218.81.55 | 0.059 |
| hzCD19_1218.81.64 | 0.033 |
| hzCD19_1218.81.79 | 0.030 |

### EXAMPLE 8: Preparation of Lentivirus Comprising Chimeric Antigen Receptor Conjugated with Affinity-Improved and Humanized Antibody Fragment

Of the developed antibodies, three variants (hzCD19_1218.81, hzCD19_1218.82, and hzCD19_1218.81.79) different in affinity were used to develop chimeric antigen receptors. For a chimeric antigen receptor, codon optimization was made of a CD8 leader, an scFv-type antibody, a hinge and transmembrane domain of CD8, a cytoplasmic domain of CD137, and a cytoplasmic domain of CD3 zeta by using GeneOptimizer (Invitrogen) algorithm. The optimized sequences were digested with SpeI/PacI and ligated to pLenti6.3/V5-TOPO lentiviral vector (Invitrogen, USA) in which the promotor had been modified into EF-1 alpha. The constructs thus obtained were identified by base sequencing.

Each of the prepared lentiviral constructs was transduced, together with the plasmid pCMV-dR8.91 comprising viral coat protein VSV-G (vesicular stomatitis indiana virus G protein), gag, pol, and rev genes, into Lenti-X 293T cells (Takara Bio Inc., Japan). Transduction was performed using Lipofectamine 2000 (Invitrogen, USA) according to the manufacturer's protocol. The cell culture containing lentivirus were enriched with Lenti-X concentrator (Takara Bio Inc., Japan) and stored.

### EXAMPLE 9: Preparation and Activity of Cytotoxic T Cell Presenting on Surface thereof Chimeric Antigen Receptor Conjugated with Affinity-Improved and Humanized Antibody Fragment

Cytotoxic T cells presenting the CD19_12.18 antibody fragment (scFv)-bearing chimeric antigen receptor on the surface thereof were prepared using the lentivirus obtained in Example 8 in the same manner as in Example 5. The cytotoxic T cells presenting the chimeric antigen receptor on the surface thereof were used to examine whether the activation of the chimeric antigen receptor T cells is induced with the recognition of CD19 on cell surfaces.

Briefly, GFP-luciferase-expressing lentivirus was transduced into CD19-positive RaJi cells to construct RaJi-Luc cells which were then used in experiments. First, RaJi-Luc cells were seeded at a density of 3x10⁴ cells/well into roundbottom 96-well plates. To the RaJi-Luc cells (T)-seeded plates, the prepared cytotoxic T cells (E) were added at a predetermined treatment rate per well (T:E = 1:2, 1:5, or 1:10), followed by incubation at 37°C for 24 hours in a 5% CO₂ atmosphere. Thereafter, interferon gamma secreted to the medium was quantitated using an ELISA kit according to the manufacturer's protocol. Toxicity of cytotoxic T cells was identified through luciferase measurement (Bio-Glo Luciferase assay system, Promega, USA).

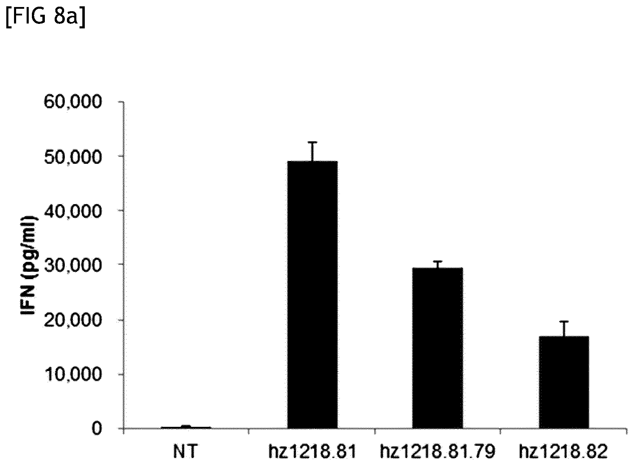

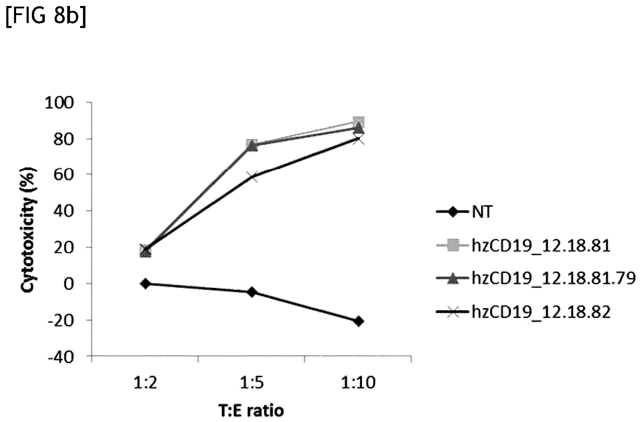

As can be seen in FIG. 8a, significant increases in the secretion of interferon gamma were detected in the experimental group treated with cytotoxic T cells (E) containing the antibody fragment of the present disclosure and the RaJi-Luc cells (T). After the cytotoxic T cells and RaJi-Luc cells were incubated together, luciferase was eluted by lysing the residual RaJi-Luc cells with 3X lysis buffer (75mM Tris(pH 8.0), 30% glycerol, 3% Triton X100) and reacted with a substrate to examine the cytotoxic effect of the chimeric antigen receptor bearing the antibody fragment of the present disclosure. Percentages of lysis were determined relative to the signal detected in the well where only Raji-Luc cells were cultured. The chimeric antigen receptor T cells having the antibody fragment of the present disclosure increased in cytotoxicity with increasing of the treatment rate. Higher cytotoxic effects were detected in cytotoxic T cells having antibody fragments better in affinity than CD19_12.18 (FIG. 8b).

### EXAMPLE 10: Development of Chimeric Antigen Receptor through Modification of Hinge Region, Transmembrane Domain, and Costimulatory Domain

In order to optimize the activity of chimeric antigen receptors employing the developed antibody fragments, new chimeric antigen receptors (CAR2 to CAR7) were developed by modifying the constituents of chimeric antigen receptors, that is, hinge regions (CD8, CD28, and Fc), transmembrane domains (CD8, CD28, and ICOS), and costimulatory domains (CD137, CD28, ICOS, and CS3). As an antibody fragment binding to CD19 antigen, hzCD19_1218.81 was employed to identify activity (FIG. 9). For the chimeric antigen receptor in each of CAR1 to CAR7, digestion and ligation to pLenti6.3/V5-TOPO lentiviral vector (Invitrogen, USA) in which the promotor had been modified into EF-1 alpha were conducted in the same manner as in Example 8. The constructs thus obtained were identified by base sequencing. Amino acid and nucleotide sequences of the constructs of CAR1 to CAR7 are set forth as SEQ ID NOS: 74 to 87 in the appended sequence listing. Each of the developed constructs was used to prepare and enrich lentivirus according to the protocol of Example 8.

The developed chimeric antigen receptors were analyzed for activity. In this regard, cytotoxic T cells were prepared in the same manner as in Example 4 and examined to see whether or not the activity of CD19-expressing cells was specifically induced.

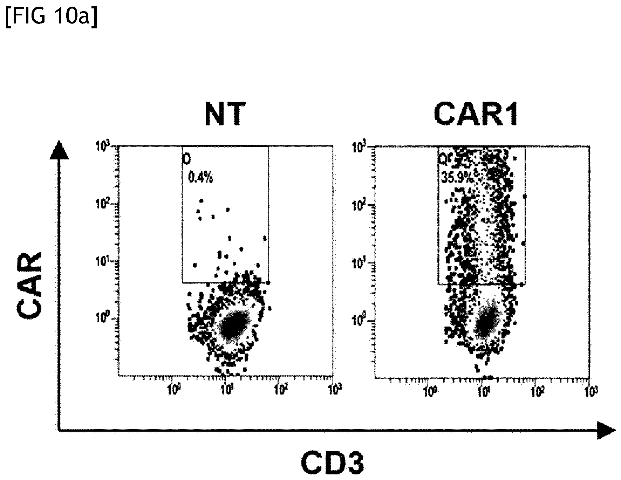

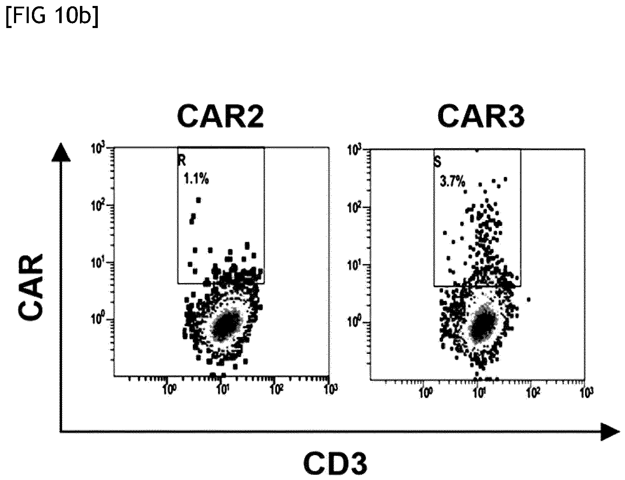

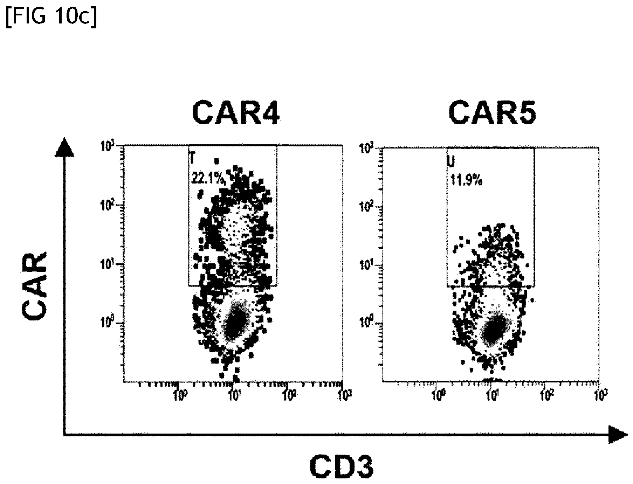

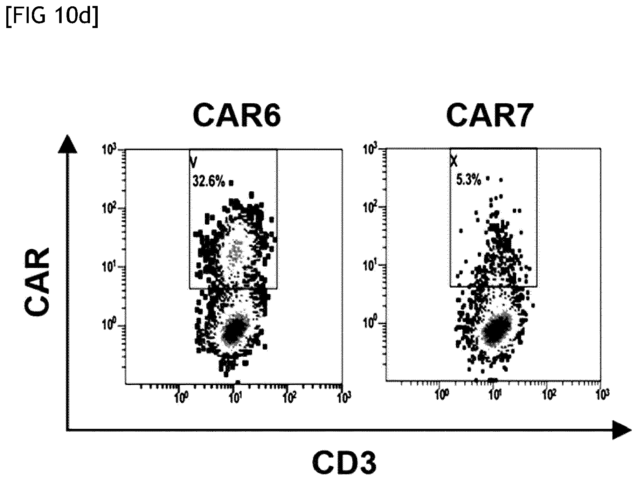

First, the obtained cytotoxic T cells were examined for CAR expression behavior. The expression of the chimeric antigen receptor was observed with the secondary antibody anti-human IgG FITC (Invitrogen, A11013) following primary binding of CD19-ECD. In this context, in order to examine whether the detected cells would be T cells or not, anti-human CD3 PE (Biolegend, 317308) was allowed to simultaneously participate in the binding, followed by flow cytometry. As a result of the assay, it was observed that the constructs (CAR2, CAR3) in which hinge region change occurred from CD8 to CD28 or Fc greatly decreased in CAR expression, compared to a construct employing a conventional CD8 hinge. In addition, the constructs in which the transmembrane domain and the costimulatory domain were changed were observed to decrease in CAR expression, compared to the case employing ICOS transmembrane domain and costimulatory domain (CAR5) (FIG. 10).

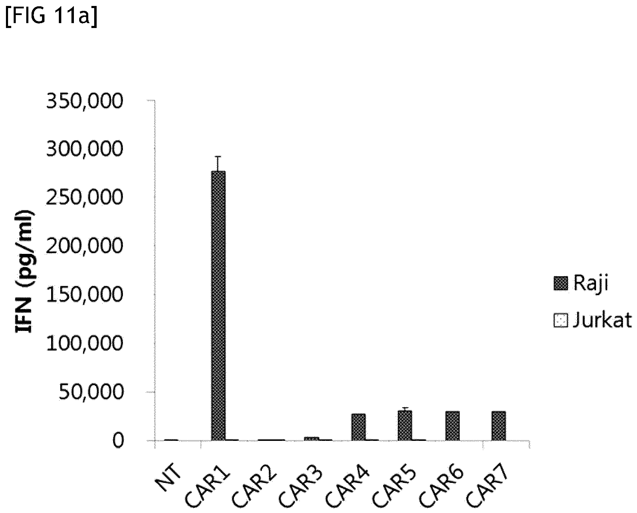

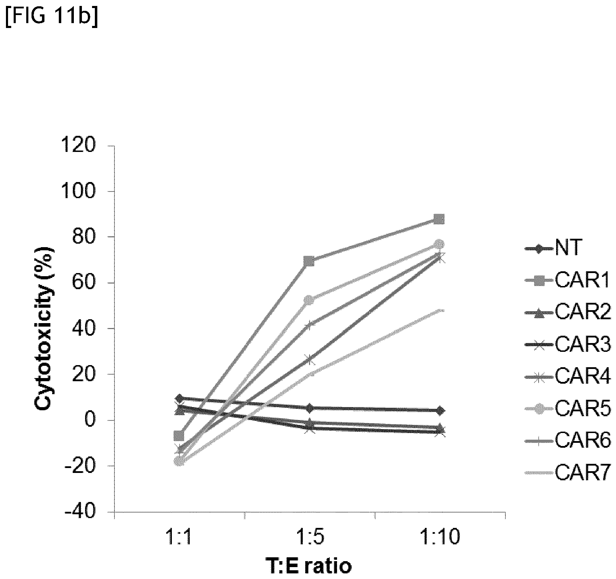

The developed cytotoxic T cells were examined for activity in the same manner as in Example 8. CD19 positive RaJi-Luc cells and CD19 negative Jurkat cells were incubated, together with cytotoxic T cells, for 24 hours, and the cell cultures were measured for interferon gamma level and cytotoxicity. As shown in FIG. 11a, an increased level of interferon gamma was detected only in the group in which CD19 positive RaJi-Luc and cytotoxic T cells were co-cultured. Furthermore, construct CAR1, which showed the best expression among the CAR constructs used in the test, induced the highest interferon gamma secretion. Unlike interferon gamma secretion, cytotoxic effects were almost evenly high in all of the constructs CAR1, CAR4, CAR5, CAR6 (FIG. 11b).

### EXAMPLE 11: Analysis of Epitope for CD19_1218 and Affinity-Improved Antibody

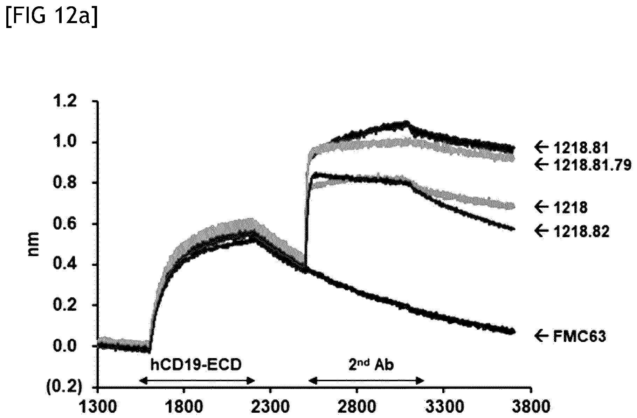

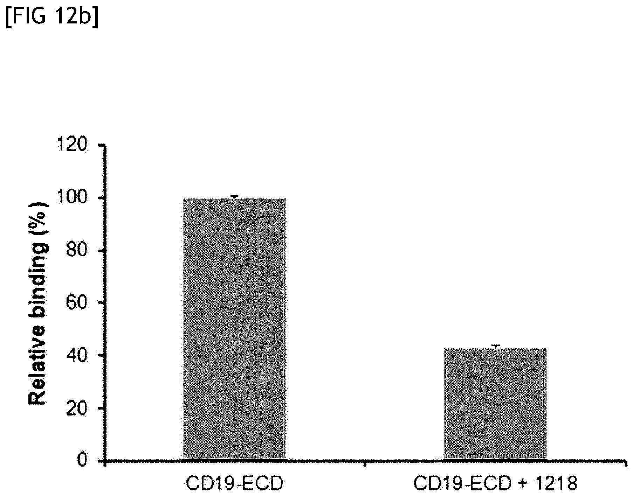

To analyze whether the CD19_1218 antibody and the affinity-improved and humanized antibodies therefrom developed in the present disclosure had an epitope in common with each other, epitope binning and competition ELISA were conducted. As described in Example 2, CD19-ECD protein was bound to FMC63 antibody-immobilized sensor chip to which FMC63, CD19_1218, hzCD19 1218.81, hzCD19 1218.81.79, and hzCD19 1218.82 antibodies were then further applied (FIG. 12a). FMC63 did not further bind to the chip whereas the four antibodies including CD19_1218 did. For competition ELISA, an ELISA plate was coated with CD19_1218.81-Fc antibody at a concentration of 2 µg/mL to which CD19-ECD-Ck (3 µg/mL) was added alone or in combination with CD19_1218-Fc antibody (300 µg/mL). Subsequently, the CD19_1218.81-Fc-bound CD19-ECD-Ck protein was quantitated using an anti-Ck-HRP antibody. The presence of CD19_1218 antibody suppressed the binding of CD19_1218.81-Fc to CD19-ECD-Ck protein (FIG. 12b). Taken together, the data obtained above demonstrate that the developed antibodies have an epitope in common with CD19_1218 antibody.

## Claims

1. A CD19-specific chimeric antigen receptor, comprising:
(a) an extracellular domain comprising a humanized anti-CD19 antibody or an antigen-binding fragment thereof;
(b) a transmembrane domain; and
(c) an intracellular signaling domain,
wherein the anti-CD19 antibody or the antigen-binding fragment thereof comprises a heavy chain variable region and a light chain variable region comprising, respectively:
(i) the sequences of SEQ ID NOS: 7 and 8;
(ii) the sequences of SEQ ID NOS: 42 and 43;
(iii) the sequences of SEQ ID NOS: 46 and 47;
(iv) the sequences of SEQ ID NOS: 50 and 51;
(v) the sequences of SEQ ID NOS: 54 and 55;
(vi) the sequences of SEQ ID NOS: 58 and 59;
(vii) the sequences of SEQ ID NOS: 62 and 63;
(viii) the sequences of SEQ ID NOS: 66 and 67; or
(ix) the sequences of SEQ ID NOS: 70 and 71.

2. An effector cell, expressing the chimeric antigen receptor of claim 1.

3. A pharmaceutical composition comprising the effector cell of claim 2 for preventing or treating a B cell malignancy selected from the group consisting of chronic lymphocytic leukemia (CLL), acute lymphocytic leukemia (ALL), pro-lymphocytic leukemia, hairy cell leukemia, common acute lymphocytic leukemia (CALLA), null-acute lymphoblastic leukemia, non Hodgkin's lymphoma, diffuse large B cell lymphoma (DLBCL), multiple myeloma, follicular lymphoma, splenic lymphoma, marginal zone lymphoma, mantle cell lymphoma, indolent B cell lymphoma, and Hodgkin's lymphoma. an autoimmune disease, or an inflammatory disease.

4. A nucleic acid molecule, encoding the chimeric antigen receptor of claim 1.

5. A recombinant vector, comprising the nucleic acid molecule of claim 4.

## Patentansprüche

1. CD19-spezifischer chimärer Antigenrezeptor, der umfasst:
(a) eine extrazelluläre Domäne, die einen humanisierten Anti-CD19-Antikörper oder ein Antigen-bindendes Fragment davon umfasst;
(b) eine Transmembrandomäne; und
(c) eine intrazelluläre Signaldomäne, wobei der Anti-CD19-Antikörper oder das Antigen-bindende Fragment davon eine variable Region der schweren Kette und eine variable Region der leichten Kette umfasst, die jeweils Folgendes umfassen:
(i) die Sequenzen von SEQ ID NOS: 7 und 8;
(ii) die Sequenzen von SEQ ID NOS: 42 und 43;
(iii) die Sequenzen von SEQ ID NOS: 46 und 47;
(iv) die Sequenzen von SEQ ID NOS: 50 und 51;
(v) die Sequenzen von SEQ ID NOS: 54 und 55;
(vi) die Sequenzen von SEQ ID NOS: 58 und 59;
(vii) die Sequenzen von SEQ ID NOS: 62 und 63;
(viii) die Sequenzen von SEQ ID NOS: 66 und 67 oder
(ix) die Sequenzen von SEQ ID NOS: 70 und 71.

2. Effektorzelle, die den chimären Antigenrezeptor nach Anspruch 1 exprimiert.

3. Pharmazeutische Zusammensetzung, die die Effektorzelle nach Anspruch 2 umfasst, zur Vorbeugung oder Behandlung einer B-Zell-Malignität, ausgewählt aus der Gruppe bestehend aus chronischer lymphatischer Leukämie (CLL), akuter lymphatischer Leukämie (ALL), prolymphatischer Leukämie, Haarzell-Leukämie, gewöhnlicher akuter lymphatischer Leukämie (CALLA), Null-akuter lymphoblastischer Leukämie, Non-Hodgkin-Lymphom, diffusem großzelligem B-Zell-Lymphom (DLBCL), Multiplem Myelom, follikulärem Lymphom, Milzlymphom, Marginalzonenlymphom, Mantelzelllymphom, indolentem B-Zell-Lymphom und Hodgkin-Lymphom, einer Autoimmunerkrankung oder einer entzündlichen Erkrankung.

4. Nukleinsäuremolekül, das den chimären Antigenrezeptor nach Anspruch 1 codiert.

5. Rekombinanter Vektor, der das Nukleinsäuremolekül nach Anspruch 4 umfasst.

## Revendications

1. Récepteur antigénique chimérique spécifique à CD19, comprenant :
(a) un domaine extracellulaire comprenant un anticorps anti-CD19 humanisé ou un fragment de liaison à l'antigène de celui-ci,
(b) un domaine transmembranaire, et
(c) un domaine de signalisation intracellulaire ;
l'anticorps anti-CD19 ou le fragment de liaison à l'antigène de celui-ci comprenant une région variable de chaîne lourde et une région variable de chaîne légère comprenant respectivement :
(i) les séquences de SEQ ID N° : 7 et 8,
(ii) les séquences de SEQ ID N° : 42 et 43,
(iii) les séquences de SEQ ID N° : 46 et 47,
(iv) les séquences de SEQ ID N° : 50 et 51,
(v) les séquences de SEQ ID N° : 54 et 55,
(vi) les séquences de SEQ ID N° : 58 et 59,
(vii) les séquences de SEQ ID N° : 62 et 63,
(viii) les séquences de SEQ ID N° : 66 et 67, ou
(ix) les séquences de SEQ ID N° : 70 et 71.

2. Cellule effectrice exprimant le récepteur antigénique chimérique selon la revendication 1.

3. Composition pharmaceutique comprenant la cellule effectrice selon la revendication 2, destinée à prévenir ou à traiter une tumeur maligne à lymphocytes B choisie dans le groupe constitué par la leucémie lymphoïde chronique (LLC), la leucémie lymphoblastique aiguë (LLA), la leucémie pro-lymphocytaire, la leucémie à cellules chevelues, la leucémie lymphoblastique aiguë commune (CALLA), la leucémie lymphoblastique aiguë de type null, le lymphome non hodgkinien, le lymphome diffus à grandes cellules B (DLBCL), le myélome multiple, le lymphome folliculaire, le lymphome splénique, le lymphome de la zone marginale, le lymphome à cellules du manteau, le lymphome indolent à cellules B et le lymphome de Hodgkin, une maladie auto-immune ou une maladie inflammatoire.

4. Molécule d'acide nucléique codant pour le récepteur antigénique chimérique selon la revendication 1.

5. Vecteur recombinant comprenant la molécule d'acide nucléique selon la revendication 4.
